# EUROPEAN PATENT APPLICATION

(11) **EP 4 846 920 A2**
(43) Date of publication of application: **02.12.2026**
(21) Application number: 26212191.6
(22) Date of filing: 27.09.2022
(51) Int. Cl.: A61B 1/267

(54) **NAVIGATION ASSISTANCE FOR AN INSTRUMENT**

(30) Priority: 28.09.2021 US 202163249440 P
(62) Document number(s) of the earlier application(s) in accordance with Art. 76 EPC: 22801915.4 / 4 408 327
(71) Applicant: Intuitive Surgical Operations, Inc., Sunnyvale, CA 94086 (US)
(72) Inventor: MOLLER, Zachary, F., Sunnyvale, 94086 (US); ADEBAR, Troy, K., Sunnyvale, 94086 (US); JENSEN, Gavin, S., Sunnyvale, 94086 (US); MULLER, Leah, R., Sunnyvale, 94086 (US); WARMAN, Cynthia, Sunnyvale, 94086 (US); WALKER, Julie, Sunnyvale, 94086 (US)
(74) Representative: J A Kemp LLP

(57) **Abstract**

Systems and methods for providing navigation indicators for an elongate device include a system configured to determine a pose of the elongate device within a passageway; and based on the pose of the elongate device, display on the display system: an image of the passageway, and one or more navigation indicators associated with one or more directions within a workspace containing the passageway, wherein the one or more navigation indicators are displayed over the image of the passageway.

## Description

### RELATED APPLICATIONS

This application claims the benefit to U.S. Provisional Application No. 63/249,440, filed September 28, 2021, and entitled "Navigation Assistance for an Instrument," which is incorporated by reference herein.

### BACKGROUND

### Field of the Various Embodiments

The present disclosure is directed to systems and methods for conducting an image-guided procedure, and more particularly to navigation assistance for an instrument during an image-guided procedure.

### Description of the Related Art

Minimally invasive medical techniques are intended to reduce the amount of tissue that is damaged during medical procedures, thereby reducing patient recovery time, discomfort, and harmful side effects. Such minimally invasive techniques may be performed through natural orifices in a patient anatomy or through one or more surgical incisions. Through these natural orifices or incisions an operator may insert minimally invasive medical instruments (including surgical, diagnostic, therapeutic, or biopsy instruments) to reach a target tissue location. One such minimally invasive technique is to use a flexible and/or steerable elongate device, such as a catheter, that can be inserted into anatomic passageways and navigated toward a region of interest within the patient anatomy. Control of such an elongate device by medical personnel during an image-guided procedure involves the management of several degrees of freedom including at least the management of insertion and retraction of the elongate device as well as steering and/or bend radius of the device. In addition, different modes of operation may also be supported.

Similar image-guided procedures can be found in non-medical contexts as well. For example, an elongate device and/or other instruments could be used to inspect and/or perform operations within pipes, ventilation shafts, passageways, enclosed spaces, and/or the like where direct access by the human operator is not possible or is not practical.

Navigation of the instrument in the passageways can be difficult. The instrument presents a limited field of view of the passageway to the operator. Accordingly, the operator can have difficulty determining an orientation of the instrument within the passageway and/or relative to the workspace where the passageway is located. If the workspace includes numerous similar-looking curves, bends, and/or junctions in the passageways, the operator can quickly become confused and disoriented while navigating the instrument, resulting in unnecessary bodily invasion and/or unnecessary and inefficient backtracking.

Accordingly, it would be advantageous to provide more effective navigation assistance for an elongate device or other instrument during an image-guided procedure.

### SUMMARY

Consistent with some embodiments, a system includes an elongate device, a display system, one or more processors, and memory storing instructions. When executed by the one or more processors, the instructions cause the one or more processors to determine a pose of the elongate device within a passageway; and based on the pose of the elongate device, display on the display system: an image of the passageway, and one or more navigation indicators associated with one or more directions within a workspace containing the passageway, wherein the one or more navigation indicators are displayed over the image of the passageway.

Consistent with some embodiments, an apparatus includes one or more processors, and memory storing instructions. When executed by the one or more processors, the instructions cause the one or more processors to determine a pose of an elongate device within a passageway; and based on the pose of the elongate device, cause to display on a display system: an image of the passageway, and one or more navigation indicators associated with one or more directions within a workspace containing the passageway, wherein the one or more navigation indicators are displayed over the image of the passageway.

Consistent with some embodiments, a system includes an elongate device, a display system, one or more processors, and memory storing instructions. When executed by the one or more processors, the instructions cause the one or more processors to cause to display on a display system: a first virtual representation of a passageway based on a pose of an elongate device, and a virtual representation of a path associated with the elongate device; receive a first input via a first control device; in response to the first input while the elongate device is being navigated in a linking mode, cause to display on the display system: a second virtual representation of the passageway corresponding to movement along the virtual representation of the path, wherein the movement along the virtual representation of the path is based on the first input; receive a second input via a second control device; and in response to the second input, cause to display on the display system: a third virtual representation of the passageway corresponding to a change to the second virtual representation along at least one of a translational or rotational degree of freedom, wherein the change to the second virtual representation is based on the second input.

Consistent with some embodiments, a method includes determining a pose of an elongate device within a passageway; and based on the pose of the elongate device, causing to be displayed on a display system: an image of the passageway, and one or more navigation indicators associated with one or more directions within a workspace containing the passageway, wherein the one or more navigation indicators are displayed over the image of the passageway.

Consistent with some embodiments, a method includes causing to be displayed on a display system: a first virtual representation of a passageway based on a pose of an elongate device, and a virtual representation of a path associated with the elongate device; receiving a first input via a first control device; in response to the first input while the elongate device is being navigated in a linking mode, causing to be displayed on the display system: a second virtual representation of the passageway corresponding to movement along the virtual representation of the path, wherein the movement along the virtual representation of the path is based on the first input; receiving a second input via a second control device; and in response to the second input, causing to be displayed on the display system: a third virtual representation of the passageway corresponding to a change to the second virtual representation along at least one of a translational or rotational degree of freedom, wherein the change to the second virtual representation is based on the second input.

Consistent with some embodiments, one or more non-transitory machine-readable media include a plurality of machine-readable instructions which when executed by one or more processors are adapted to cause the one or more processors to perform any of the methods described herein.

At least one advantage and technical improvement of the disclosed techniques relative to the prior art is that, with the disclosed techniques, navigational assistance for an elongate device is provided in a clearer and more informative manner to an operator relative to conventional approaches. The navigational assistance can be used with live image-based guidance and/or dynamic registration guidance. Accordingly, an operator of the elongate device can more effectively navigate the elongate device within one or more passageways (e.g., within a patient) with reduced likelihood of navigating down an undesired path and undesired backtracking. Another advantage and technical improvement is that, while changes to the pose of an elongate device (e.g., movement of the elongate device) is linked to movement along a planned path in dynamic registration guidance, a virtual view on the planned path can be adjusted to better match a live view from the elongate device without causing a change in the pose of the elongate device. Accordingly, an operator can manually adjust a virtual view in dynamic registration guidance when the virtual view does not match the live view. These technical advantages provide one or more technological advancements over prior art approaches.

### BRIEF DESCRIPTION OF THE DRAWINGS

So that the manner in which the above recited features of the various embodiments can be understood in detail, a more particular description of the inventive concepts, briefly summarized above, may be had by reference to various embodiments, some of which are illustrated in the appended drawings. It is to be noted, however, that the appended drawings illustrate only typical embodiments of the inventive concepts and are therefore not to be considered limiting of scope in any way, and that there are other equally effective embodiments.
FIG. 1 is a simplified diagram of a teleoperated medical system according to some embodiments.
FIG. 2A is a simplified diagram of a medical instrument system according to some embodiments.
FIG. 2B is a simplified diagram of a medical instrument system with an extended medical instrument according to some embodiments.
FIGS. 3A and 3B are simplified diagrams of side views of a patient coordinate space including a medical instrument mounted on an insertion assembly according to some embodiments.
FIG. 4 is a simplified diagram of a graphical user interface displayable on a display system according to some embodiments.
FIG. 5 is a simplified diagram of an example local view according to some embodiments.
FIG. 6 is a simplified diagram of another example local view according to some embodiments.
FIGs. 7A-7B are diagrams of an example orientation guide indicating different elongate device poses, according to some embodiments.
FIGs. 8A-8C are simplified diagrams of a user interface having a live camera view and a virtual view at different times, according to some embodiments.
FIG. 8D is a simplified diagram of a user interface having a live camera view, a virtual view, a tree view, and a navigational view, according to some embodiments.
FIG. 9 is a simplified diagram of a graphical user interface for manually adjusting a virtual view, according to some embodiments.
FIG. 10 is a flow chart of method steps for providing navigation guidance for an elongate device, according to some embodiments.
FIGs. 11A-11B are a flow chart of method steps for adjusting a virtual representation, according to some embodiments.

### DETAILED DESCRIPTION

In the following description, specific details are set forth describing some embodiments consistent with the present disclosure. Numerous specific details are set forth in order to provide a thorough understanding of the embodiments. It will be apparent, however, to one skilled in the art that some embodiments may be practiced without some or all of these specific details. The specific embodiments disclosed herein are meant to be illustrative but not limiting. One skilled in the art may realize other elements that, although not specifically described here, are within the scope and the spirit of this disclosure. In addition, to avoid unnecessary repetition, one or more features shown and described in association with one embodiment may be incorporated into other embodiments unless specifically described otherwise or if the one or more features would make an embodiment non-functional.

Further, the terminology in this description is not intended to limit the invention. For example, spatially relative terms-such as "beneath", "below", "lower", "above", "upper", "proximal", "distal", and the like-may be used to describe the relation of one element or feature to another element or feature as illustrated in the figures. These spatially relative terms are intended to encompass different positions and orientations of the elements or their operation in addition to the position and orientation shown in the figures. For example, if the content of one of the figures is turned over, elements described as "below" or "beneath" other elements or features would then be "above" or "over" the other elements or features. Thus, the illustrative term "below" can encompass both positions and orientations of above and below. A device may be otherwise oriented (rotated 90 degrees or at other orientations) and the spatially relative descriptors used herein interpreted accordingly. Likewise, descriptions of movement along and around various axes include various special element positions and orientations. In addition, the singular forms "a", "an", and "the" are intended to include the plural forms as well, unless the context indicates otherwise. And, the terms "comprises", "comprising", "includes", and the like specify the presence of stated features, steps, operations, elements, and/or components but do not preclude the presence or addition of one or more other features, steps, operations, elements, components, and/or groups. Components described as coupled may be electrically or mechanically directly coupled, or they may be indirectly coupled via one or more intermediate components.

Elements described in detail with reference to one embodiment, implementation, or module may, whenever practical, be included in other embodiments, implementations, or modules in which they are not specifically shown or described. For example, if an element is described in detail with reference to one embodiment and is not described with reference to a second embodiment, the element may nevertheless be claimed as included in the second embodiment. Thus, to avoid unnecessary repetition in the following description, one or more elements shown and described in association with one embodiment, implementation, or application may be incorporated into other embodiments, implementations, or aspects unless specifically described otherwise, unless the one or more elements would make an embodiment or implementation non-functional, or unless two or more of the elements provide conflicting functions.

In some instances, well known methods, procedures, components, and circuits have not been described in detail so as not to unnecessarily obscure aspects of the embodiments.

This disclosure describes various instruments and portions of instruments in terms of their state in three-dimensional space. As used herein, the term "position" refers to the location of an object or a portion of an object in a three-dimensional space (e.g., three degrees of translational freedom along Cartesian x-, y-, and z-coordinates). As used herein, the term "orientation" refers to the rotational placement of an object or a portion of an object (three degrees of rotational freedom - e.g., roll, pitch, and yaw). As used herein, the term "pose" refers to the position of an object or a portion of an object in at least one degree of translational freedom and to the orientation of that object or portion of the object in at least one degree of rotational freedom (up to six total degrees of freedom). As used herein, the term "shape" refers to a set of poses, positions, or orientations measured along an object.

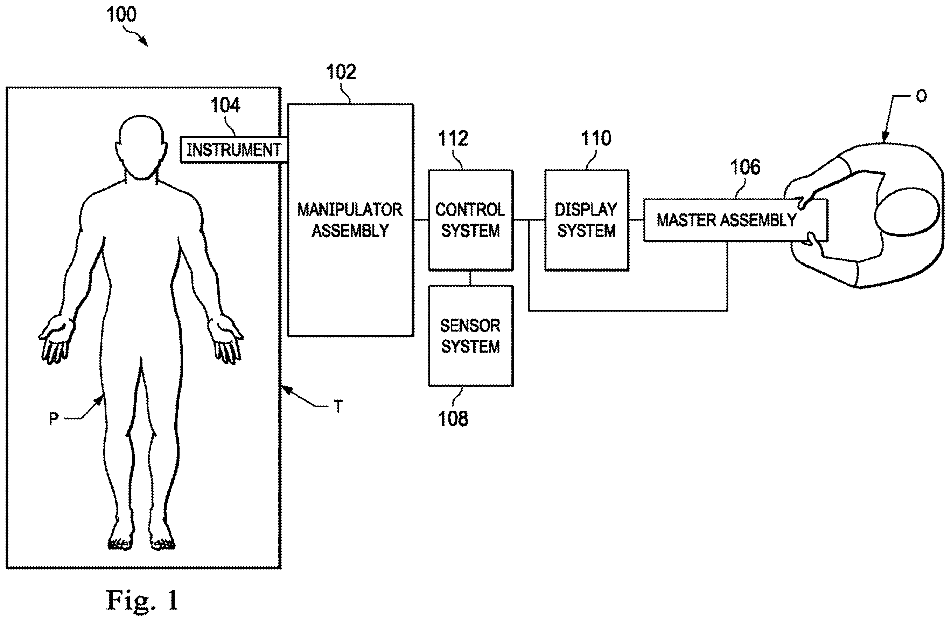

FIG. 1 is a simplified diagram of a teleoperated medical system 100 according to some embodiments. In some embodiments, teleoperated medical system 100 may be suitable for use in, for example, surgical, diagnostic, therapeutic, or biopsy procedures. While some embodiments are provided herein with respect to such procedures, any reference to medical or surgical instruments and medical or surgical methods is non-limiting. The systems, instruments, and methods described herein may be used for animals, human cadavers, animal cadavers, portions of human or animal anatomy, non-surgical diagnosis, as well as for industrial systems and general robotic or teleoperational systems.

As shown in FIG. 1, medical system 100 generally includes a manipulator assembly 102 for operating a medical instrument 104 in performing various procedures on a patient P. The manipulator assembly 102 may be teleoperated, non-teleoperated, or a hybrid teleoperated and non-teleoperated assembly with select degrees of freedom of motion that may be motorized and/or teleoperated and select degrees of freedom of motion that may be non-motorized and/or non- teleoperated. Manipulator assembly 102 is mounted to or near an operating table T. A master assembly 106 allows an operator O (e.g., a surgeon, a clinician, or a physician as illustrated in FIG. 1) to view the interventional site and to control manipulator assembly 102.

Master assembly 106 may be located at an operator console which is usually located in the same room as operating table T, such as at the side of a surgical table on which patient P is located. However, it should be understood that operator O can be located in a different room or a completely different building from patient P. Master assembly 106 generally includes one or more control devices for controlling manipulator assembly 102. The control devices may include any number of a variety of input devices, such as joysticks, trackballs, scroll wheels, data gloves, trigger-guns, hand- operated controllers, voice recognition devices, body motion or presence sensors, and/or the like. To provide operator O a strong sense of directly controlling instruments 104 the control devices may be provided with the same degrees of freedom as the associated medical instrument 104. In this manner, the control devices provide operator O with telepresence or the perception that the control devices are integral with medical instruments 104.

In some embodiments, the control devices may have more or fewer degrees of freedom than the associated medical instrument 104 and still provide operator O with telepresence. In some embodiments, the control devices may optionally be manual input devices which move with six degrees of freedom, and which may also include an actuatable handle for actuating instruments (for example, for closing grasping jaws, applying an electrical potential to an electrode, delivering a medicinal treatment, and/or the like).

Manipulator assembly 102 supports medical instrument 104 and may include a kinematic structure of one or more non-servo controlled links (e.g., one or more links that may be manually positioned and locked in place, generally referred to as a set-up structure), and/or one or more servo controlled links (e.g., one more links that may be controlled in response to commands from the control system), and a manipulator. Manipulator assembly 102 may optionally include a plurality of actuators or motors that drive inputs on medical instrument 104 in response to commands from the control system (e.g., a control system 112). The actuators may optionally include drive systems that when coupled to medical instrument 104 may advance medical instrument 104 into a naturally or surgically created anatomic orifice. Other drive systems may move the distal end of medical instrument 104 in multiple degrees of freedom, which may include three degrees of linear motion (e.g., linear motion along the X, Y, Z Cartesian axes) and in three degrees of rotational motion (e.g., rotation about the X, Y, Z Cartesian axes). Additionally, the actuators can be used to actuate an articulable end effector of medical instrument 104 for grasping tissue in the jaws of a biopsy device and/or the like. Actuator position sensors such as resolvers, encoders, potentiometers, and other mechanisms may provide sensor data to medical system 100 describing the rotation and orientation of the motor shafts. This position sensor data may be used to determine motion of the objects manipulated by the actuators.

Teleoperated medical system 100 may include a sensor system 108 with one or more sub-systems for receiving information about the instruments of manipulator assembly 102. Such sub-systems may include a position/location sensor system (e.g., an electromagnetic (EM) sensor system); a shape sensor system for determining the position, orientation, speed, velocity, pose, and/or shape of a distal end and/or of one or more segments along a flexible body that may make up medical instrument 104; and/or a visualization system for capturing images from the distal end of medical instrument 104.

Teleoperated medical system 100 also includes a display system 110 for displaying an image or representation of the surgical site and medical instrument 104 generated by sub-systems of sensor system 108. Display system 110 and master assembly 106 may be oriented so operator O can control medical instrument 104 and master assembly 106 with the perception of telepresence.

In some embodiments, medical instrument 104 may have a visualization system (discussed in more detail below), which may include a viewing scope assembly that records a concurrent or real-time image of a surgical site and provides the image to the operator or operator O through one or more displays of medical system 100, such as one or more displays of display system 110. The concurrent image may be, for example, a two or three dimensional image captured by an endoscope positioned within the surgical site. In some embodiments, the visualization system includes endoscopic components that may be integrally or removably coupled to medical instrument 104. However in some embodiments, a separate endoscope, attached to a separate manipulator assembly may be used with medical instrument 104 to image the surgical site. The visualization system may be implemented as hardware, firmware, software or a combination thereof which interact with or are otherwise executed by one or more computer processors, which may include the processors of a control system 112.

Display system 110 may also display an image of the surgical site and medical instruments captured by the visualization system. In some examples, teleoperated medical system 100 may configure medical instrument 104 and controls of master assembly 106 such that the relative positions of the medical instruments are similar to the relative positions of the eyes and hands of operator O. In this manner operator O can manipulate medical instrument 104 and the hand control as if viewing the workspace in substantially true presence. By true presence, it is meant that the presentation of an image is a true perspective image simulating the viewpoint of a physician that is physically manipulating medical instrument 104.

In some examples, display system 110 may present images of a surgical site recorded pre-operatively or intra-operatively using image data from imaging technology such as, computed tomography (CT), magnetic resonance imaging (MRI), fluoroscopy, thermography, ultrasound, optical coherence tomography (OCT), thermal imaging, impedance imaging, laser imaging, nanotube X-ray imaging, and/or the like. The preoperative or intra-operative image data may be presented as two-dimensional, three-dimensional, or four-dimensional (including e.g., time based or velocity based information) images and/or as images from models created from the pre-operative or intra-operative image data sets.

In some embodiments, often for purposes of imaged guided surgical procedures, display system 110 may display a virtual navigational image in which the actual location of medical instrument 104 is registered (e.g., dynamically referenced) with the pre-operative or concurrent images/model. This may be done to present the operator O with a virtual image of the internal surgical site from a viewpoint of medical instrument 104. In some examples, the viewpoint may be from a tip of medical instrument 104. An image of the tip of medical instrument 104 and/or other graphical or alphanumeric indicators may be superimposed on the virtual image to assist operator O controlling medical instrument 104. In some examples, medical instrument 104 may not be visible in the virtual image.

In some embodiments, display system 110 may display a virtual navigational image in which the actual location of medical instrument 104 is registered with preoperative or concurrent images to present the operator O with a virtual image of medical instrument 104 within the surgical site from an external viewpoint. An image of a portion of medical instrument 104 or other graphical or alphanumeric indicators may be superimposed on the virtual image to assist operator O in the control of medical instrument 104. As described herein, visual representations of data points may be rendered to display system 110. For example, measured data points, moved data points, registered data points, and other data points described herein may be displayed on display system 110 in a visual representation. The data points may be visually represented in a user interface by a plurality of points or dots on display system 110 or as a rendered model, such as a mesh or wire model created based on the set of data points. In some examples, the data points may be color coded according to the data they represent. In some embodiments, a visual representation may be refreshed in display system 110 after each processing operation has been implemented to alter data points.

Teleoperated medical system 100 may also include control system 112. Control system 112 includes at least one memory and at least one computer processor (not shown) for effecting control between medical instrument 104, master assembly 106, sensor system 108, and display system 110. Control system 112 also includes programmed instructions (e.g., a non-transitory machine-readable medium storing the instructions) to implement some or all of the methods described in accordance with aspects disclosed herein, including instructions for providing information to display system 110. While control system 112 is shown as a single block in the simplified schematic of FIG. 1, the system may include two or more data processing circuits with one portion of the processing optionally being performed on or adjacent to manipulator assembly 102, another portion of the processing being performed at master assembly 106, and/or the like. The processors of control system 112 may execute instructions comprising instruction corresponding to processes disclosed herein and described in more detail below. Any of a wide variety of centralized or distributed data processing architectures may be employed. Similarly, the programmed instructions may be implemented as a number of separate programs or subroutines, or they may be integrated into a number of other aspects of the teleoperational systems described herein. In one embodiment, control system 112 supports wireless communication protocols such as Bluetooth, IrDA, HomeRF, IEEE 802.11, DECT, and Wireless Telemetry.

In some embodiments, control system 112 may receive force and/or torque feedback from medical instrument 104. Responsive to the feedback, control system 112 may transmit signals to master assembly 106. In some examples, control system 112 may transmit signals instructing one or more actuators of manipulator assembly 102 to move medical instrument 104. Medical instrument 104 may extend into an internal surgical site within the body of patient P via openings in the body of patient P. Any suitable conventional and/or specialized actuators may be used. In some examples, the one or more actuators may be separate from, or integrated with, manipulator assembly 102. In some embodiments, the one or more actuators and manipulator assembly 102 are provided as part of a teleoperational cart positioned adjacent to patient P and operating table T.

Control system 112 may optionally further include a virtual visualization system to provide navigation assistance to operator O when controlling medical instrument 104 during an image-guided surgical procedure. Virtual navigation using the virtual visualization system may be based upon reference to an acquired preoperative or intraoperative dataset of anatomic passageways. The virtual visualization system processes images of the surgical site imaged using imaging technology such as computerized tomography (CT), magnetic resonance imaging (MRI), fluoroscopy, thermography, ultrasound, optical coherence tomography (OCT), thermal imaging, impedance imaging, laser imaging, nanotube X-ray imaging, and/or the like. Software, which may be used in combination with manual inputs, is used to convert the recorded images into segmented two-dimensional or three-dimensional composite representation of a partial or an entire anatomic organ or anatomic region. An image data set is associated with the composite representation. The composite representation and the image data set describe the various locations and shapes of the passageways and their connectivity. The images used to generate the composite representation may be recorded preoperatively or intra-operatively during a clinical procedure. In some embodiments, a virtual visualization system may use standard representations (e.g., not patient specific) or hybrids of a standard representation and patient specific data. The composite representation and any virtual images generated by the composite representation may represent the static posture of a deformable anatomic region during one or more phases of motion (e.g., during an inspiration/ expiration cycle of a lung).

During a virtual navigation procedure, sensor system 108 may be used to compute an approximate location of medical instrument 104 with respect to the anatomy of patient P. The location can be used to produce both macro-level (external) tracking images of the anatomy of patient P and virtual internal images of the anatomy of patient P. The system may implement one or more electromagnetic (EM) sensor, fiber optic sensors, and/or other sensors to register and display a medical implement together with preoperatively recorded surgical images, such as those from a virtual visualization system. For example, PCT Publication WO 2016/191298 (published December 1, 2016) (disclosing "Systems and Methods of Registration for Image Guided Surgery"), which is incorporated by reference herein in its entirety, discloses such one system. Teleoperated medical system 100 may further include optional operations and support systems (not shown) such as illumination systems, steering control systems, irrigation systems, and/or suction systems. In some embodiments, teleoperated medical system 100 may include more than one manipulator assembly and/or more than one master assembly. The exact number of teleoperational manipulator assemblies will depend on the surgical procedure and the space constraints within the operating room, among other factors. Master assembly 106 may be collocated or they may be positioned in separate locations. Multiple master assemblies allow more than one operator to control one or more teleoperational manipulator assemblies in various combinations.

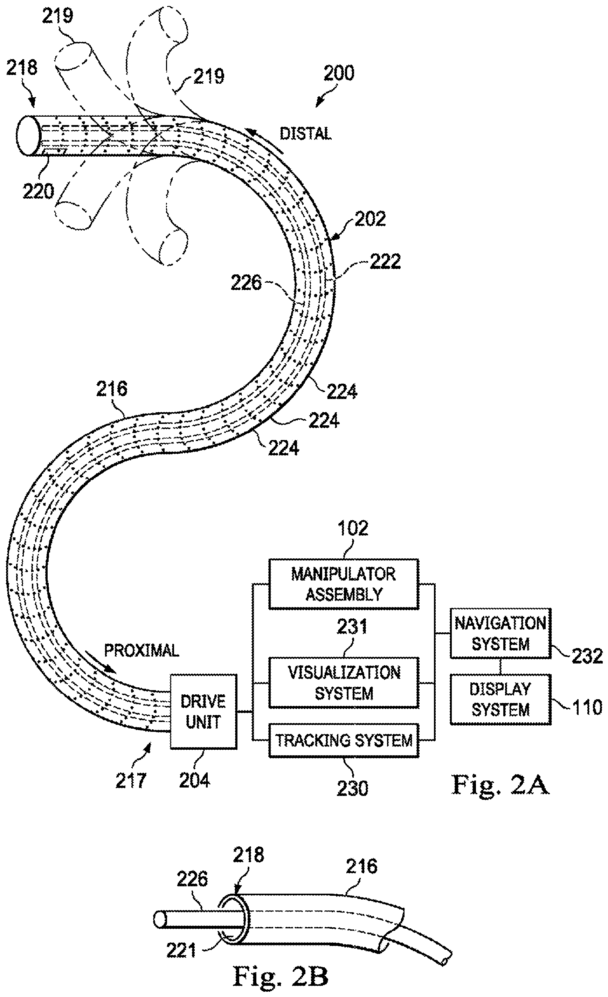

FIG. 2A is a simplified diagram of a medical instrument system 200 according to some embodiments. In some embodiments, medical instrument system 200 may be used as medical instrument 104 in an image-guided medical procedure performed with teleoperated medical system 100. In some examples, medical instrument system 200 may be used for non-teleoperational exploratory procedures or in procedures involving traditional manually operated medical instruments, such as endoscopy. Optionally medical instrument system 200 may be used to gather (e.g., measure) a set of data points corresponding to locations within anatomic passageways of a patient, such as patient P.

Medical instrument system 200 includes elongate device 202, such as a flexible catheter, coupled to a drive unit 204. Elongate device 202 includes a flexible body 216 having proximal end 217 and a distal end 218. In some embodiments, flexible body 216 has an approximately 3 mm outer diameter. Other flexible body outer diameters may be larger or smaller.

Medical instrument system 200 further includes a tracking system 230 for determining the position, orientation, speed, velocity, pose, and/or shape of distal end 218 and/or of one or more segments 224 along flexible body 216 using one or more sensors and/or imaging devices as described in further detail below. The entire length of flexible body 216, between distal end 218 and proximal end 217, may be effectively divided into segments 224. Tracking system 230 may optionally be implemented as hardware, firmware, software or a combination thereof which interact with or are otherwise executed by one or more computer processors, which may include the processors of control system 112 in FIG. 1.

Tracking system 230 may optionally track distal end 218 and/or one or more of the segments 224 using a shape sensor 222. Shape sensor 222 may optionally include an optical fiber aligned with flexible body 216 (e.g., provided within an interior channel (not shown) or mounted externally). In one embodiment, the optical fiber has a diameter of approximately 200 pm. In other embodiments, the dimensions may be larger or smaller. The optical fiber of shape sensor 222 forms a fiber optic bend sensor for determining the shape of flexible body 216. In one alternative, optical fibers including Fiber Bragg Gratings (FBGs) are used to provide strain measurements in structures in one or more dimensions. Various systems and methods for monitoring the shape and relative position of an optical fiber in three dimensions are described in U.S. Patent Application Publication No. 2006/0013523 (filed July 13, 2005) (disclosing "Fiber optic position and shape sensing device and method relating thereto"); U.S. Patent No. 7,772,541 (filed on Jul. 16, 2004) (disclosing "Fiber-optic shape and relative position sensing"); and U.S. Patent No. 6,389,187 (filed on Jun. 17, 1998) (disclosing "Optical Fibre Bend Sensor"), which are all incorporated by reference herein in their entireties. Sensors in some embodiments may employ other suitable strain sensing techniques, such as Rayleigh scattering, Raman scattering, Brillouin scattering, and Fluorescence scattering. In some embodiments, the shape of the elongate device may be determined using other techniques. For example, a history of the distal end pose of flexible body 216 can be used to reconstruct the shape of flexible body 216 over the interval of time. In some embodiments, tracking system 230 may optionally and/or additionally track distal end 218 using a position sensor system 220. Position sensor system 220 may be a component of an EM sensor system with position sensor system 220 including one or more conductive coils that may be subjected to an externally generated electromagnetic field. Each coil of the EM sensor system then produces an induced electrical signal having characteristics that depend on the position and orientation of the coil relative to the externally generated electromagnetic field. In some embodiments, position sensor system 220 may be configured and positioned to measure six degrees of freedom, e.g., three position coordinates X, Y, Z and three orientation angles indicating pitch, yaw, and roll of a base point or five degrees of freedom, e.g., three position coordinates X, Y, Z and two orientation angles indicating pitch and yaw of a base point. Further description of a position sensor system is provided in U.S. Patent No. 6,380,732 (filed August 11, 1999) (disclosing "Six-Degree of Freedom Tracking System Having a Passive Transponder on the Object Being Tracked"), which is incorporated by reference herein in its entirety.

In some embodiments, tracking system 230 may alternately and/or additionally rely on historical pose, position, or orientation data stored for a known point of an instrument system along a cycle of alternating motion, such as breathing. This stored data may be used to develop shape information about flexible body 216. In some examples, a series of positional sensors (not shown), such as electromagnetic (EM) sensors similar to the sensors in position sensor system 220 may be positioned along flexible body 216 and then used for shape sensing. In some examples, a history of data from one or more of these sensors taken during a procedure may be used to represent the shape of elongate device 202, particularly if an anatomic passageway is generally static.

Flexible body 216 includes a channel 221 sized and shaped to receive a medical instrument 226. FIG. 2B is a simplified diagram of flexible body 216 with medical instrument 226 extended according to some embodiments. In some embodiments, medical instrument 226 may be used for procedures such as surgery, biopsy, ablation, illumination, irrigation, or suction. Medical instrument 226 can be deployed through channel 221 of flexible body 216 and used at a target location within the anatomy. Medical instrument 226 may include, for example, image capture probes, biopsy instruments, laser ablation fibers, and/or other surgical, diagnostic, or therapeutic tools. Medical tools may include end effectors having a single working member such as a scalpel, a blunt blade, an optical fiber, an electrode, and/or the like. Other end effectors may include, for example, forceps, graspers, scissors, clip appliers, and/or the like. Other end effectors may further include electrically activated end effectors such as electro surgical electrodes, transducers, sensors, and/or the like. In various embodiments, medical instrument 226 is a biopsy instrument, which may be used to remove sample tissue or a sampling of cells from a target anatomic location. Medical instrument 226 may be used with an image capture probe also within flexible body 216. In various embodiments, medical instrument 226 may be an image capture probe that includes a distal portion with a stereoscopic or monoscopic camera at or near distal end 218 of flexible body 216 for capturing images (including video images) that are processed by a visualization system 231 for display and/or provided to tracking system 230 to support tracking of distal end 218 and/or one or more of the segments 224. The image capture probe may include a cable coupled to the camera for transmitting the captured image data. In some examples, the image capture instrument may be a fiber-optic bundle, such as a fiberscope, that couples to visualization system 231. The image capture instrument may be single or multi- spectral, for example capturing image data in one or more of the visible, infrared, and/or ultraviolet spectrums. Alternatively, medical instrument 226 may itself be the image capture probe. Medical instrument 226 may be advanced from the opening of channel 221 to perform the procedure and then retracted back into the channel when the procedure is complete. Medical instrument 226 may be removed from proximal end 217 of flexible body 216 or from another optional instrument port (not shown) along flexible body 216.

Medical instrument 226 may additionally house cables, linkages, or other actuation controls (not shown) that extend between its proximal and distal ends to controllably the bend distal end of medical instrument 226. Steerable instruments are described in detail in U.S. Patent No. 7,316,681 (filed on Oct. 4, 2005) (disclosing "Articulated Surgical Instrument for Performing Minimally Invasive Surgery with Enhanced Dexterity and Sensitivity") and U.S. Patent No. 9,259,274 (filed Sept. 30, 2008) (disclosing "Passive Preload and Capstan Drive for Surgical Instruments"), which are incorporated by reference herein in their entireties.

Flexible body 216 may also house cables, linkages, or other steering controls (not shown) that extend between drive unit 204 and distal end 218 to controllably bend distal end 218 as shown, for example, by broken dashed line depictions 219 of distal end 218. In some examples, at least four cables are used to provide independent "up-down" steering to control a pitch of distal end 218 and "left-right" steering to control a yaw of distal end 281. Steerable elongate devices are described in detail in U.S. Patent No. 9,452,276 (filed Oct. 14, 2011) (disclosing "Catheter with Removable Vision Probe"), which is incorporated by reference herein in its entirety. In embodiments in which medical instrument system 200 is actuated by a teleoperational assembly, drive unit 204 may include drive inputs that removably couple to and receive power from drive elements, such as actuators, of the teleoperational assembly. In some embodiments, medical instrument system 200 may include gripping features, manual actuators, or other components for manually controlling the motion of medical instrument system 200. Elongate device 202 may be steerable or, alternatively, the system may be non-steerable with no integrated mechanism for operator control of the bending of distal end 218. In some examples, one or more lumens, through which medical instruments can be deployed and used at a target surgical location, are defined in the walls of flexible body 216.

In some embodiments, medical instrument system 200 may include a flexible bronchial instrument, such as a bronchoscope or bronchial catheter, for use in examination, diagnosis, biopsy, or treatment of a lung. Medical instrument system 200 is also suited for navigation and treatment of other tissues, via natural or surgically created connected passageways, in any of a variety of anatomic systems, including the colon, the intestines, the kidneys and kidney calices, the brain, the heart, the circulatory system including vasculature, and/or the like.

The information from tracking system 230 may be sent to a navigation system 232 where it is combined with information from visualization system 231 and/or the preoperatively obtained models to provide the physician or other operator with real-time position information. In some examples, the real-time position information may be displayed on display system 110 of FIG. 1 for use in the control of medical instrument system 200. In some examples, control system 116 of FIG. 1 may utilize the position information as feedback for positioning medical instrument system 200. Various systems for using fiber optic sensors to register and display a surgical instrument with surgical images are provided in U.S. Patent No. 8,900,131, filed May 13, 2011, disclosing "Medical System Providing Dynamic Registration of a Model of an Anatomic Structure for Image-Guided Surgery," and PCT Publication WO 2016/191298, filed May 20, 2016, disclosing "Systems and Methods of Registration for Image Guided Surgery," which are incorporated by reference herein in their entirety.

In some examples, medical instrument system 200 may be teleoperated within medical system 100 of FIG. 1. In some embodiments, manipulator assembly 102 of FIG. 1 may be replaced by direct operator control. In some examples, the direct operator control may include various handles and operator interfaces for hand-held operation of the instrument.

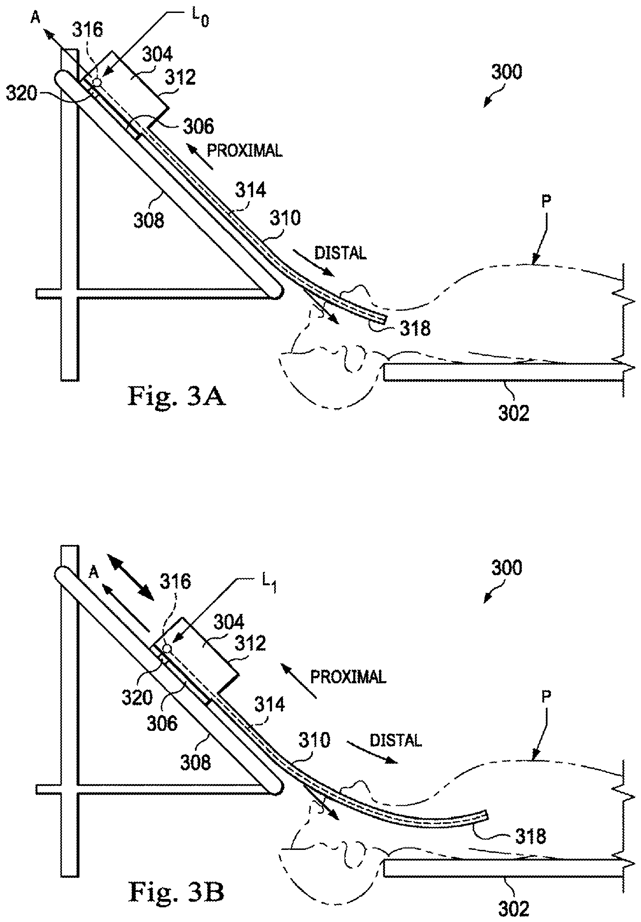

FIGS. 3A and 3B are simplified diagrams of side views of a patient coordinate space including a medical instrument mounted on an insertion assembly according to some embodiments. As shown in FIGS. 3A and 3B, a surgical environment 300 includes a patient P that is positioned on the table T of FIG. 1. Patient P may be stationary within the surgical environment in the sense that gross patient movement is limited by sedation, restraint, and/or other means. Cyclic anatomic motion including respiration and cardiac motion of patient P may continue, unless the patient is asked to hold his or her breath to temporarily suspend respiratory motion. Accordingly, in some embodiments, data may be gathered at a specific, phase in respiration, and tagged and identified with that phase. In some embodiments, the phase during which data is collected may be inferred from physiological information collected from patient P. Within surgical environment 300, a point gathering instrument 304 is coupled to an instrument carriage 306. In some embodiments, point gathering instrument 304 may use EM sensors, shape- sensors, and/or other sensor modalities. Instrument carriage 306 is mounted to an insertion stage 308 fixed within surgical environment 300. Alternatively, insertion stage 308 may be movable but have a known location (e.g., via a tracking sensor or other tracking device) within surgical environment 300. Instrument carriage 306 may be a component of a manipulator assembly (e.g., manipulator assembly 102) that couples to point gathering instrument 304 to control insertion motion (e.g., motion along the A axis) and, optionally, motion of a distal end 318 of an elongate device 310 in multiple directions including yaw, pitch, and roll. Instrument carriage 306 or insertion stage 308 may include actuators, such as servomotors, (not shown) that control motion of instrument carriage 306 along insertion stage 308.

Elongate device 310 is coupled to an instrument body 312. Instrument body 312 is coupled and fixed relative to instrument carriage 306. In some embodiments, an optical fiber shape sensor 314 is fixed at a proximal point 316 on instrument body 312. In some embodiments, proximal point 316 of optical fiber shape sensor 314 may be movable along with instrument body 312 but the location of proximal point 316 may be known (e.g., via a tracking sensor or other tracking device). Shape sensor 314 measures a shape from proximal point 316 to another point such as distal end 318 of elongate device 310. Point gathering instrument 304 may be substantially similar to medical instrument system 200.

A position measuring device 320 provides information about the position of instrument body 312 as it moves on insertion stage 308 along an insertion axis A. Position measuring device 320 may include resolvers, encoders, potentiometers, and/or other sensors that determine the rotation and/or orientation of the actuators controlling the motion of instrument carriage 306 and consequently the motion of instrument body 312. In some embodiments, insertion stage 308 is linear. In some embodiments, insertion stage 308 may be curved or have a combination of curved and linear sections.

FIG. 3A shows instrument body 312 and instrument carriage 306 in a retracted position along insertion stage 308. In this retracted position, proximal point 316 is at a position L₀ on axis A. In this position along insertion stage 308 an A component of the location of proximal point 316 may be set to a zero and/or another reference value to provide a base reference to describe the position of instrument carriage 306, and thus proximal point 316, on insertion stage 308. With this retracted position of instrument body 312 and instrument carriage 306, distal end 318 of elongate device 310 may be positioned just inside an entry orifice of patient P. Also in this position, position measuring device 320 may be set to a zero and/or the another reference value (e.g., 1=0). In FIG. 3B, instrument body 312 and instrument carriage 306 have advanced along the linear track of insertion stage 308 and distal end 318 of elongate device 310 has advanced into patient P. In this advanced position, the proximal point 316 is at a position L₁ on the axis A. In some examples, encoder and/or other position data from one or more actuators controlling movement of instrument carriage 306 along insertion stage 308 and/or one or more position sensors associated with instrument carriage 306 and/or insertion stage 308 is used to determine the position L₁ of proximal point 316 relative to position L₀. In some examples, position L₁ may further be used as an indicator of the distance or insertion depth to which distal end 318 of elongate device 310 is inserted into the passageways of the anatomy of patient P.

In an illustrative application, a medical instrument system, such as medical instrument system 200, may include a robotic catheter system for use in lung biopsy procedures. A catheter of the robotic catheter system provides a conduit for tools such as endoscopes, endobronchial ultrasound (EBUS) probes, therapeutic tools, and/or biopsy tools to be delivered to locations within the airways where one or more targets of the lung biopsy, such as lesions, nodules, tumors, and/or the like, are present. When the catheter is driven through anatomy, typically an endoscope is installed such that a clinician, such as operator O, can monitor a live camera view of a distal end of the catheter. The live camera view and/or other real-time navigation information may be displayed to the clinician via a graphical user interface.

Before a biopsy procedure is performed using the robotic catheter system, preoperative planning steps may be performed to plan the biopsy procedure. Pre-operative planning steps may include segmentation of a patient CT scan to create a three-dimensional (3D) representation (e.g., a 3D model) of anatomy, selecting targets within the 3D model, determining airways in the model, growing the airways to form a connected tree of airways, and planning a path to the targets through the connected tree. One or more of these steps may be performed on the same robotic catheter system used to perform the biopsy, on a different medical instrument system, on a standalone processor, such as a workstation dedicated to preoperative planning, and/or the like. The plan for the biopsy procedure may be saved (e.g., as one or more digital files) and transferred to the robotic catheter system used to perform the biopsy procedure. The saved plan may include the 3D model, identification of airways, target locations, paths to target locations, and/or the like. An example of a graphical user interface supporting the pre-operative planning steps is covered in U.S. Patent Application Publication No. 2020/0030044, entitled "Graphical User Interface for Planning a Procedure" and filed on September 20, 2019, which is incorporated by reference in its entirety.

After the plan is transferred to the robotic catheter system, the 3D model of the anatomy may be registered to the actual patient anatomy and/or the catheter within the patient anatomy. Consequently, the real-time position and orientation of the catheter may be projected onto the 3D model and displayed via the graphical user interface. The clinician can then proceed with driving the catheter through anatomy while monitoring navigation progress on the graphical user interface. For example, the clinician may drive the catheter along a predetermined path in the saved plan to navigate to the target location and/or perform a biopsy at a target location.

Illustrative embodiments of a graphical user interface for monitoring a medical procedure, including but not limited to the lung biopsy procedure described above, are provided below. The graphical user interface may include a registration mode that is used to monitor the registration of a 3D model to an anatomy, a navigation mode that is used to monitor the navigation of a medical instrument to a target location in the anatomy, and a performance mode that is used to monitor the performance of an interventional step at the target location. Some aspects of the graphical user interface are similar to features described in International Patent Application Publication No. WO 2018/005861, entitled "Graphical User Interface for Displaying Guidance Information During an Image-Guided Procedure" and filed Jun. 29, 2017, and International Patent Application Publication No. WO 2018/005842, entitled "Graphical User Interface for Displaying Guidance Information in a Plurality of Modes During an Image-Guided Procedure" and filed Jun. 29, 2017, which are hereby incorporated by reference in their entirety.

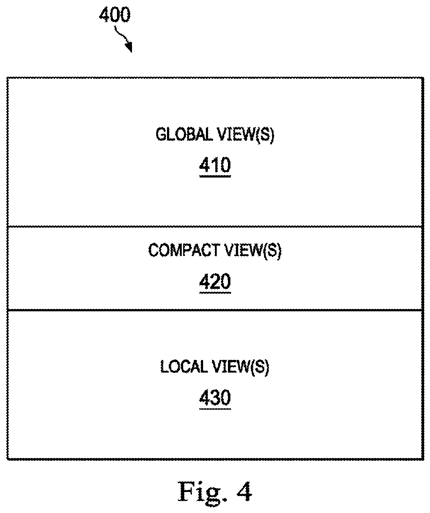

FIG. 4 is a simplified diagram of a graphical user interface 400 displayable on a display system, such as display system 110, according to some embodiments. Graphical user interface 400 displays information associated with a medical procedure in one or more views that are viewable to a clinician, such as operator O. Although an illustrative arrangement of views is depicted in FIG. 4, it is to be understood that graphical user interface 400 may display any suitable number of views, in any suitable arrangement, and/or on any suitable number of screens. In some examples, the number of concurrently displayed views may be varied by opening and closing views, minimizing and maximizing views, moving views between a foreground and background of graphical user interface 400, switching between screens, and/or otherwise fully or partially obscuring views. Similarly, the arrangement of the views-including their size, shape, orientation, ordering (in a case of overlapping views), and/or the like-may vary and/or may be user-configurable.

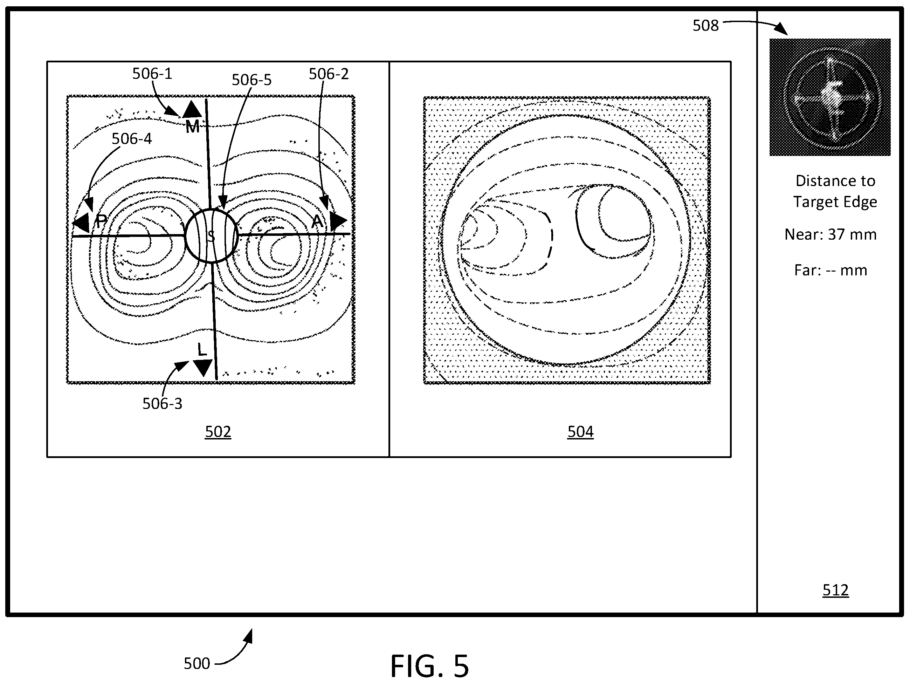

In some examples, the views displayed in graphical user interface 400 may be arranged in an organized scheme to facilitate rapid access to relevant information. Although FIG. 4 depicts an illustrative example of one such organization scheme, many other organization schemes are possible. As depicted in FIG. 4, graphical user interface 400 includes an upper portion that displays one or more global views 410, a middle portion that displays one or more compact views 420, and a lower portion that displays one or more local views 430. Global views 410 generally display global aspects of the medical procedure to provide the clinician with a detailed picture of the current state of the medical procedure. Compact views 420 generally display a reduced set of information about the medical procedure in a simplified, uncluttered format to facilitate rapid comprehension by the clinician. Local views 430 generally display local aspects of the medical procedure to monitor movements and/or interventional steps performed by the medical instrument in real-time. Examples of local views 430 are discussed in greater detail below with reference to FIGS. 5-8C.

In some examples, global, compact, and local views 410-430 may be arranged in various configurations other than those depicted in FIG. 4. For example, graphical user interface 400 may have a landscape layout in which global views 410 are positioned on the left, compact views 420 are oriented vertically in the middle, and local views 430 are positioned on the right. In some examples, global, compact, and local views 410-430 may be spread throughout graphical user interface 400, such that graphical user interface 400 may not be divisible into dedicated regions as depicted in FIG. 4. In some examples, graphical user interface 400 may include various views, controls, indicators, and/or the like, in addition to those depicted in FIG. 4. For example, graphical user interface 400 may include a header, footer, one or more sidebars, message bars, popup windows, backgrounds, overlays, and/or the like.

Graphical user interface 400 may be operated in different modes at various stages of the medical procedure. In some examples, the organization scheme may vary based on the mode of graphical user interface 400. In each mode, the arrangement of views may be selected to convey information that is available and/or relevant at the current stage of the medical procedure. **In** some examples, the modes may include a registration mode, a navigation mode, and/or a performance mode as discussed below. **In** some examples, various modes may overlap with each other and/or transition seamlessly between each other so as to behave as a single mode. For example, the navigation and performance modes may be seamlessly transitioned such that they may be considered a single hybrid navigation and performance mode.

**In** some embodiments, graphical user interface 400 can further include one or more interactive user interface elements. For example, in embodiments where display system 110 includes a touch-sensitive display (e.g., a touch screen), graphical user interface 400 could include one or more user interface elements (e.g., buttons, sliders, dials, switches, toggles, and/or the like) that operator O can interact with via touches on the touch-sensitive display, and control system 112 receives the touches as input. That is, the touch-sensitive display is also a control device within medical system 100. The user interface elements could be displayed in one view that is in addition to or in place of any of global, compact, or local views 410-430. Further, in the same embodiments with the touch-sensitive display, operator O could interact with any of global, compact, and/or local views 410-430 as well.

As described above, graphical user interface 400 can operate in a navigation mode that is used to monitor the navigation of a medical instrument to a target location in the anatomy. While in the navigation mode, graphical user interface 400 can include a local view 430 that includes a live camera view of images captured by instrument 104 inside patient P. Local view 430 in navigation mode can also include a virtual view that displays a rendering of the 3D model of the anatomy of patient P (e.g., from the perspective of the distal end of instrument 104) according to a current registration. Local view 430 in navigation mode can further include one or more visual aids to provide navigational information and/or assistance to operator O as operator O navigates instrument 104 within patient P. Examples of local views in navigational mode are described below with reference to FIGs. 5-6.

FIG. 5 is a simplified diagram of an example local view according to some embodiments. Local view 500, which is in navigation mode, includes a live camera view 502 and a virtual view 504 displayed side-by-side. Live camera view 502 displays live, real-time images captured at the distal end of an elongate device (e.g., instrument 104, elongate device 202, a catheter) within a patient anatomy (e.g., patient P). Live camera view 502 can be captured by an imaging device (e.g., a camera, an endoscope) located at the distal end of the elongate device. Live camera view 502 as shown in FIG. 5 is a first-person view from the distal end of the elongate device (e.g., the endoscope camera capturing the images is located at or near the distal end of the elongate device). In some embodiments, live camera view 502 can instead be a third-person view, where the camera capturing the live images is slightly behind the distal end of the elongate device, and at least a portion of the distal end is within the field of view of the camera.

Virtual view 504 is a virtual navigational image that corresponds to a view of the surroundings of the elongate device. In some embodiments, virtual view 504 corresponds to a view from the perspective of the distal end of the elongate device. Virtual view 504 can be a rendering of a 3D model of the patient anatomy (e.g., from the perspective of the distal end of the elongate device), according to a current registration between the 3D model and the patient anatomy.

One or more navigation indicators 506 can be displayed concurrently with and over (e.g., overlaid onto) live camera view 502. Navigation indicators can include indicators of directions and/or headings (e.g., direction/heading indicator) relative to the perspective shown in live camera view 502 and/or virtual view 504. Navigation indicators 506 indicate (e.g., point towards) one or more directions or headings relative to the patient anatomy, from the perspective of live camera view 502 (and correspondingly, from the perspective of the distal end of the elongate device). In some embodiments, navigation indicators 506 indicate anatomical directions or headings, from the perspective of live camera view 502, based on a current pose (e.g., current position and/or orientation) of the elongate device relative to the patient anatomy. Accordingly, navigation indicators 506 can provide navigational information and guidance to operator O as operator O monitors the patient anatomy and navigates the elongate device through the patient anatomy.

As shown, navigation indicators 506 include a number of directions or headings that are above, below, and/or to a side of live camera view 502. Navigation indicators 506 include a pointer indicator 506-1 indicating a medial heading ("M") relative to the patient anatomy that is above live camera view 502, a pointer indicator 506-2 indicating an anterior heading ("A") relative to the patient anatomy that is to the right of live camera view 502, a pointer indicator 506-3 indicating a lateral heading ("L") relative to the patient anatomy that is below live camera view 502, and a pointer indicator 506-4 indicating a posterior heading ("P") relative to the patient anatomy that is to the left of live camera view 502. Operator O can direct live camera view 502 toward any of the headings indicated by pointer indicators 506-1 thru 506-4 by pointing the distal end of the elongate device toward the heading pointed to by any of pointer indicators 506-1 through 506-4. Similarly, Operator O can navigate the elongate device toward any of the headings indicated by pointer indicators 506-1 through 506-4 by moving the elongate device toward the heading pointed to by any of pointer indicators 506-1 through 506-4.

Navigation indicators 506 also include an indicator 506-5 indicating the superior heading ("S") relative to the patient anatomy. As shown, the superior heading is in front (e.g., straight ahead) of live camera view 502. Accordingly, indicator 506-5 is displayed as an icon displayed over an intersection of two perpendicular lines, resembling a reticle. In some embodiments, the two perpendicular lines that intersect at the icon correspond to anatomical planes (e.g., the coronal, sagittal, or transverse planes). Operator O can maintain live camera view 502 in the superior heading indicated by indicator 506-5 by continuing to point the distal end of the elongate device in the straight-ahead direction indicated by indicator 506-5. Similarly, Operator O can navigate the elongate device toward the superior heading indicated by indicator 506-5 by moving the elongate device in the straight-ahead direction indicated by indicator 506-5.

The inferior heading, being behind live camera view 502 as shown, is not indicated by an indicator 506 in live camera view 502. An operator O can infer that the inferior heading is behind live camera view 502 by the absence of an indicator 506 indicating the inferior heading. In some other embodiments, navigation indicators 506 can include an indicator that indicates a direction that is behind live camera view 502 (e.g., an additional pointer indicator similar to pointer indicators 506-1 thru 506-4). In some embodiments, indicators 506 include indicators for each of one or more (e.g., four) anatomical directions that are the closest angularly to the heading direction of the elongate device.

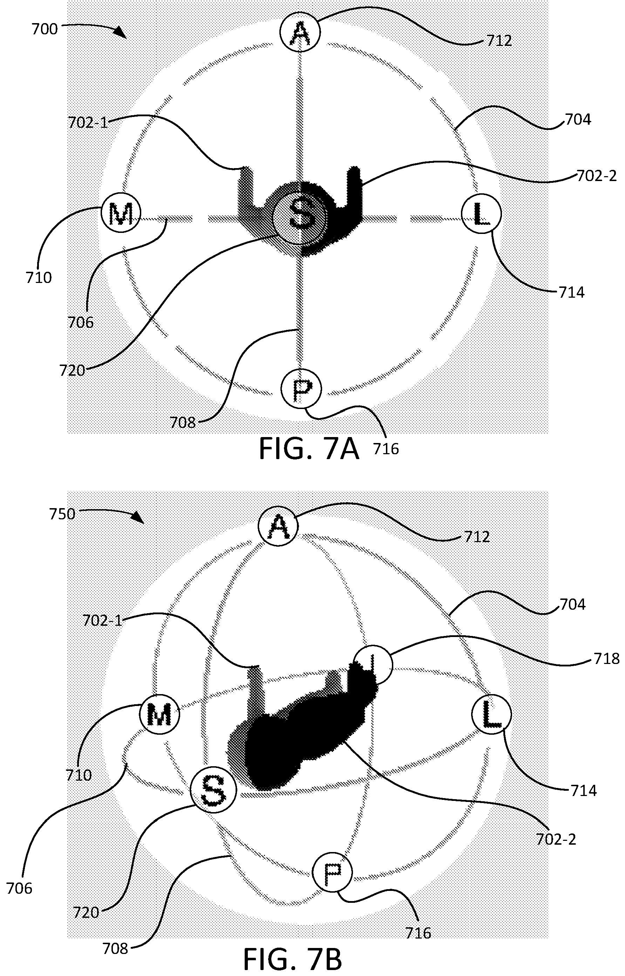

Local view 500 can also include an information sidebar 512 that displays additional information (e.g., distance to a target edge, etc.) that can be useful to operator O navigating the elongate device. As shown, information sidebar 512 includes an orientation guide 508. Orientation guide 508 provides further navigational information and guidance to operator O in the form of a graphic that indicates the pose of the elongate device, the same as live camera view 502, from a point of view that is external to the patient anatomy. Accordingly, in orientation guide 508 the direction looking into the display is the same direction as that of live camera view 502. The graphic in orientation guide 508 can include representation of a human centered within a virtual sphere. The graphic in orientation guide 508 can also include indicators of the anatomical headings and one or more equator circles corresponding to anatomical planes. Further details regarding orientation guides are described below with reference to FIG. 7A.

In various embodiments, indicators 506 and orientation guide 508, and other information displayed in information sidebar 512, can be generated and/or updated by control system 112 based on current poses of the elongate device and displayed on display system 110 (e.g., in local view 500 along with live camera view 502). As operator O navigates the elongate device, control system 112 regularly generates and/or updates indicators 506 on live camera view 502 and orientation guide 508 based on the current pose of the elongate device. Updates can include modifying where an indicator 506 is pointing toward, where an indicator 506 is on live camera view 502, adding or removing an indicator 506, and/or the like.

In some embodiments, control system 112 can automatically modify indicators 506 indicating the medial and lateral headings based on the current pose of the elongate device relative to the sagittal plane (e.g., based on whether the elongate device is in the right lung or left lung in a lung procedure). For example, when the elongate device is on the right side of the sagittal plane and pointing toward the inferior heading (e.g., when the elongate device is in the right lung in a lung procedure), the medial heading may be to the left of the elongate device and the lateral heading may be to the right of the elongate device, and indicators 506 for the medial heading and lateral heading may point accordingly. When the elongate device crosses to the left side of the sagittal plane while still pointing toward the inferior heading (e.g., when the elongate device is in the left lung in a lung procedure), the medial heading is then to the right of the elongate device and the lateral heading is to the left of the elongate device, and control system 112 can automatically update the indicators 506 for the medial heading and lateral heading (e.g., automatically swap the navigation indicator for the medial heading and the navigation indicator for the lateral heading) to account for the crossing of the sagittal plane by the elongate device.

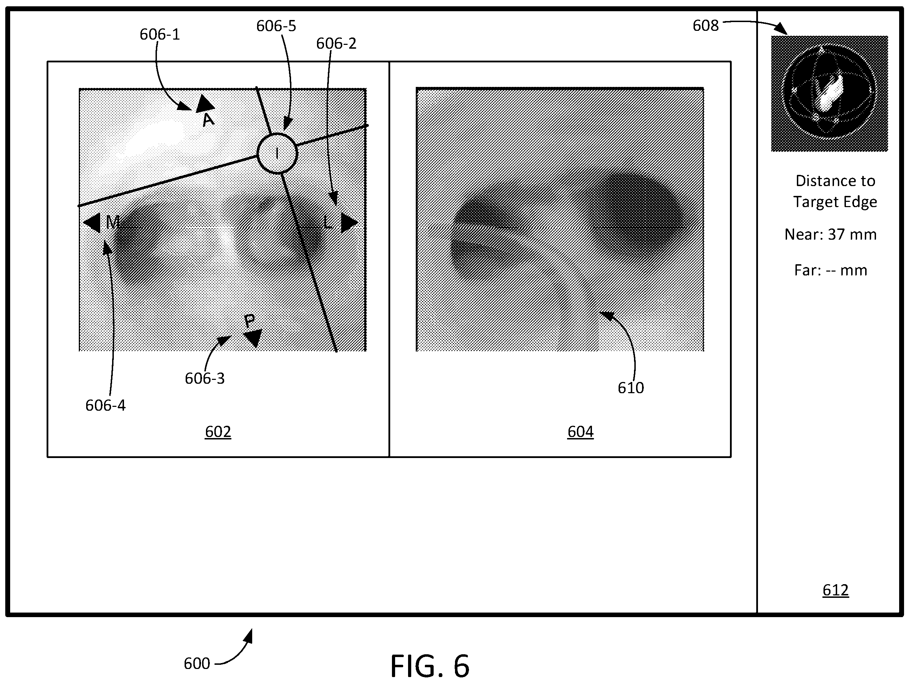

FIG. 6 is a simplified diagram of another example local view according to some embodiments. FIG. 6 illustrates a local view 600 that is similar to local view 500 of FIG. 5 in many aspects. In some embodiments, local view 600 is a local view in the same patient anatomy as and at a different time from local view 500. Similar to local view 500, local view 600 includes a live camera view 602 and virtual view 604 displayed side-by-side. Navigation indicators 606 are displayed over live camera view 602, similar to the display of navigation indicators 506 over live camera view 502. As shown, indicators 606-1, 606-2, 606-3, and 606-4 indicates the anterior heading, lateral heading, posterior heading, and medial heading, respectively. Indicator 606-5 indicates the inferior heading. No indicator is shown for the superior heading, which is behind live camera view 602. Local view 600 also includes an information sidebar 612. Information sidebar 612 includes an orientation guide 608 that indicates the pose of the elongate device, the same as live camera view 602, from a point of view that is external to the patient anatomy. Further details regarding orientation guides are described further below with reference to FIG. 7B.

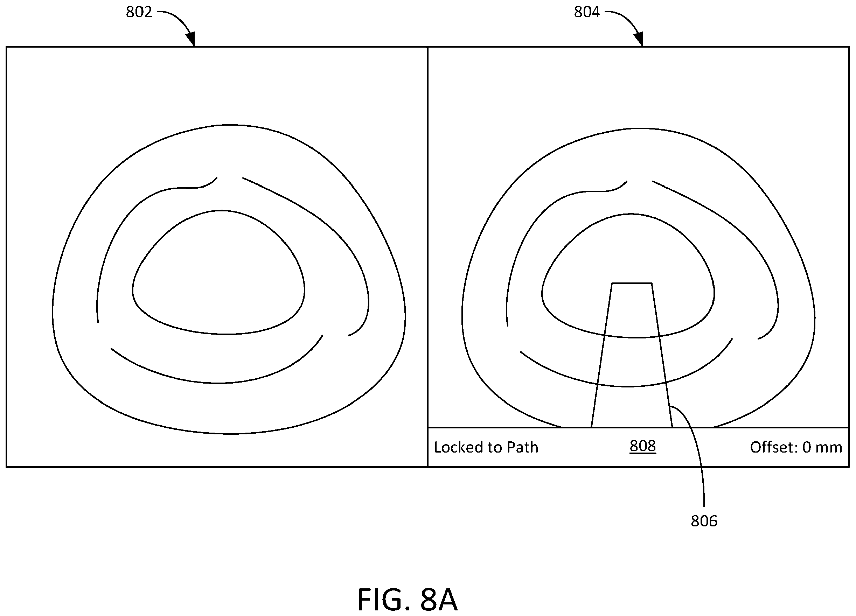

Local view 600 further includes a planned path 610 displayed concurrently with and over virtual view 604. Planned path 610 can be generated based on a saved plan (e.g., a saved pre-operative plan for a procedure). Operator O can use planned path 610 as a guide on where to navigate the elongate device within patient anatomy during the procedure to arrive at a target location associated with planned path 610. Planned paths are further described below with reference to FIGs. 8A-8C.

While FIG. 5 shows navigation indicators 506 displayed over live camera view 502, navigation indicators 506 can additionally or alternatively be displayed concurrently with and over virtual view 504. Similarly in FIG. 6, navigation indicators 606 can additionally or alternatively be displayed concurrently with and over virtual view 604 and planned path 610.

FIGs. 7A-7B illustrate an orientation guide indicating different elongate device poses, according to some embodiments. FIG. 7A shows an orientation guide 700 indicating a first pose of an elongate device. Orientation guide 700 includes a body representation 702 representing the body or anatomy of patient P. Body representation 702 can include a 3D representation of an anatomy, including representations of a head, arms, and/or legs. As shown, the arms on body representation 702 are extended toward the anterior heading to indicate the front and back sides of body representation 702. Also, consistent with the distal end of the elongate device pointing in the inferior direction of the patient anatomy, body representation 702 is oriented such that the top of the head on body representation 702 faces operator O. That is, the head on body representation 702 oriented away from the display and toward operator O viewing orientation guide 700, while the legs on body representation 702 are oriented into the display, away from operator O viewing orientation guide 700.

Within orientation guide 700, body representation 702 is centered within a virtual sphere. One or more equator lines corresponding to the anatomical planes can run along a virtual surface of the virtual sphere (e.g., at the orthogonal circumferences of the virtual sphere). As shown, orientation guide 700 includes a transverse plane equator line 704, a coronal plane equator line 706, and a sagittal plane equator line 708. The transverse, coronal, and sagittal planes intersect each other at body representation 702 as centered in the virtual sphere.

The equator lines can include heading indicators indicating the anatomical directions/headings. As shown, an anterior heading indicator 712 and a posterior heading indicator 716 are located at respective corresponding intersections of transverse plane equator line 704 and sagittal plane equator line 708. A medial heading indicator 710 and a lateral heading indicator 714 are located at respective corresponding intersections of transverse plane equator line 704 and coronal plane equator line 706. A superior heading indicator 720 is located at the corresponding intersection of coronal plane equator line 706 and sagittal plane equator line 708. Because of the first pose of the elongate device, superior heading indicator 720 is facing operator O, and an inferior heading indicator is behind body representation 702 and accordingly is at least partially obscured by body representation 702 and therefore not shown in FIG. 7A. In some embodiments, the equator lines on the virtual sphere can optionally include gaps or hash marks as additional visual aids to operator O for discerning the orientation of the elongate device. The gaps or hashes can be placed at regular angular intervals along the equator lines. For example, in FIG. 7A, each of the equator lines include gaps at the 45-degree points between the anatomical heading indicators along the equator line.

Turning to FIG. 7B, orientation guide 750 is the same orientation guide as orientation guide 700, but indicating a second, different pose of the elongate device. Orientation guide 750 includes body representation 702. As shown in FIG. 7B, the arms on body representation 702 are extended toward the anterior heading to indicate the front and back sides of body representation 702, as with FIG. 7A. Also, consistent with the distal end of the elongate device pointing toward the inferior direction, the head on body representation 702 is oriented away from the display and toward operator O viewing orientation guide 750, while the legs on body representation 702 is oriented into the display, away from operator O viewing orientation guide 750.

As with orientation guide 700, body representation 702 in orientation guide 750 is centered within the virtual sphere. Transverse plane equator line 704, coronal plane equator line 706, and sagittal plane equator line 708 run along the virtual surface of the virtual sphere, as with orientation guide 700. Orientation guide 750 also includes medial heading indicator 710, anterior heading indicator 712, lateral heading indicator 714, posterior heading indicator 716, and superior heading indicator 720, as with orientation guide 700. Further, inferior heading indicator 718 is now at least partially visible and is shown at the corresponding intersection of coronal plane equator line 706 and sagittal plane equator line 708.

As operator O navigates the elongate device within the patient anatomy, control system 112 can update orientation guide 700 or 750 based on the current pose of the elongate device. For example, as operator O navigates the elongate device from a first pose to a second pose, control system 112 can rotate the virtual sphere and body representation 702 from the orientation shown in orientation guide 700 to that shown in orientation guide 750 to track the pose of the elongate device.

In some embodiments, body representation 702 includes a highlighted half 702-1 and a shaded or darkened (or more generally not highlighted) half 702-2. The highlighted half 702-1 corresponds to and indicates a half of the patient anatomy, on one side of the sagittal plane, in which the elongate device is currently positioned. The not-highlighted half 702-2 corresponds to and indicates the other half of the patient anatomy, on the opposite side of the sagittal plane, in which the elongate device is not currently positioned. Whenever the elongate device crosses the sagittal plane from one half of the patient anatomy to the other half, control system 112 can update body representation 702 to change which half of body representation 702 is highlighted and which is not highlighted. In some embodiments, the highlighted half can be distinguished from the non-highlighted half in any suitable manner (e.g., contrasting colors and/or shades). For example, the highlighted half could be colored a lighter color or shade (e.g., white, a light shade), and the non-highlighted half could be colored a darker color or shade (e.g., black or dark gray, a dark shade). Control system 112 can further update the locations of medial heading indicator 710 and lateral heading indicator 714 (e.g., swap their locations) to correspond to the new position of the elongate device after the elongate device crosses the sagittal plane from one half of the patient anatomy to the other half.

It should be appreciated that while the embodiments disclosed in conjunction with FIGs. 5-7B are illustrated and described with reference to a medical example with anatomical directions/headings and anatomical planes, the disclosed embodiments are adaptable to non-medical contexts and another 3D coordinate frame. For example, the navigation indicators and orientation guide could indicate headings and planes in a Cartesian coordinate frame, where the headings correspond to positive or negative directions along x, y, or z axes, and the planes correspond to the x, y, or z planes. As another example, the navigation indicators and orientation guide could indicate headings and planes in a compass-based coordinate frame (e.g., the headings correspond to cardinal directions (e.g., north, south, east, west) and up or down). As a further example, in the orientation guide body representation 702 could be replaced by a different representation, shape, or icon that more appropriately represent the non-medical workspace.

FIGs. 8A-8C are simplified diagrams of a user interface having a live camera view and a virtual view at different times, according to some embodiments. FIG. 8A illustrates a live camera view 802 and a virtual view 804 side-by-side at a first time. Live camera view 802 and virtual view 804 can be displayed in a graphical user interface 400 in navigation mode, in particular a local view 430 (e.g., local view 500 or 600). Live camera view 802, similar to live camera view 502 or 602, may display live, real-time images captured at the distal end of the elongate device (e.g., by an endoscope at the distal end) within a patient anatomy. Virtual view 804 is a virtual navigational image of the patient anatomy (e.g., a view of the patient anatomy from the perspective of the distal end of the elongate device). Virtual view 804 can be a rendering of a 3D model of the patient anatomy from the perspective of the distal end of the elongate device, according to a current registration between the 3D model and the patient anatomy. Also displayed in virtual view 804 may be a planned path 806 and an information bar 808.

In some embodiments, control system 112 can, by command of operator O, link or "lock" images displayed in virtual view 804 to images along planned path 806, based on movement of the elongate device. In a linking mode, the control system 112 can automatically adjust virtual view 804 as the elongate device moves in the patient anatomy so that virtual view 804 roughly corresponds to live camera view 802. The linking mode may be helpful when, for example, registration between a 3D model and the patient anatomy is inaccurate, which may be caused by patient motion, deformation of anatomy, etc. **In** the linking mode, the control system 112 might no longer rely on the registration between the 3D model and patient anatomy to determine images to display in virtual view 804. For example, operator O may operate a control device (e.g., a scroll wheel, a trackball) at master assembly 106 to move the elongate device in the patient anatomy, such as in the insertion direction or retraction direction, and live camera view 802 may display the corresponding live images as the elongate device moves. Under the linking mode, virtual view 804 may be linked to the movement of the elongate device, e.g., in a manner corresponding to the movement along planned path 806. As the elongate device is commanded to move in patient anatomy, virtual view 804 may be updated based on the commanded movement. For example, if the operator relies on live camera view 802 and advances the elongate device in the insertion direction, virtual view 804 may be automatically advanced (e.g., by a set amount) along planned path 806 to roughly correspond to live camera view 802. Accordingly, operator O can move the elongate device by moving along planned path 806 using the control device, and virtual view 804 may be locked to planned path 806 and display virtual images corresponding to the movement of the elongate device even when, for example, registration is inaccurate.

Information bar 808 can display information including a linking mode indicator and an offset value. The linking mode indicator indicates whether virtual view 804 is linked to movement of the elongate device in the manner described above. For example, a mode or status "Locked to Path," as shown in FIG. 8A, would indicate that a linking mode is active, and accordingly virtual view 804 is linked to movement of the elongate device. If the linking mode is not active, , the linking mode or status can be omitted from display, or a mode/status indicator to that effect (e.g., "Not Locked to Path") can be displayed in information bar 808. The offset value displayed in information bar 808 is further described below.

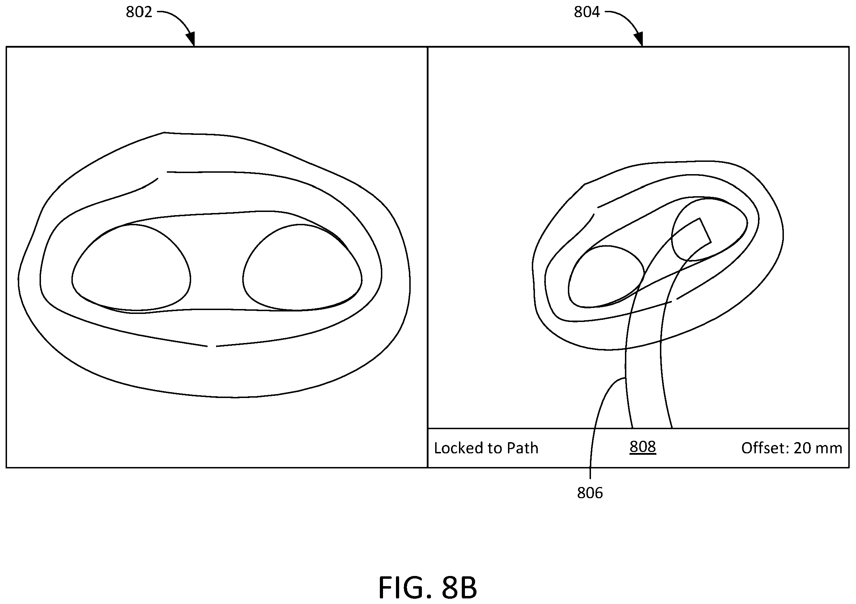

As the elongate device moves within the patient anatomy while a linking mode is active, and live camera view 802 and virtual view 804 are updated accordingly, live camera view 802 and virtual view 804 can deviate from each other. In particular, because movement of the elongate device while the linking mode is active may be interpreted into straight movement along planned path 806, any actual movement of the elongate device that deviates from planned path 806 still causes virtual view 804 to be updated as if the movement was along planned path 806, which can result in deviation between live camera view 802 and virtual view 804. Because of the deviation, live camera view 802 and virtual view 804 might not substantially match. For example, FIG. 8B illustrates live camera view 802 and virtual view 804 at a second time (e.g., after operator O has moved the elongate device within the patient anatomy some amount from the pose shown in FIG. 8A, and virtual view 804 is updated along planned path 806 based on the movement of the elongate device). While both live camera view 802 and virtual view 804 in FIG. 8B might show the same branching passageway, the branching passageway shown in virtual view 804 appears further back than the branching passageway as shown in live camera view 802. Further, the branching passageway shown in virtual view 804 appears rotated relative to the branching passageway as shown in live camera view 802.

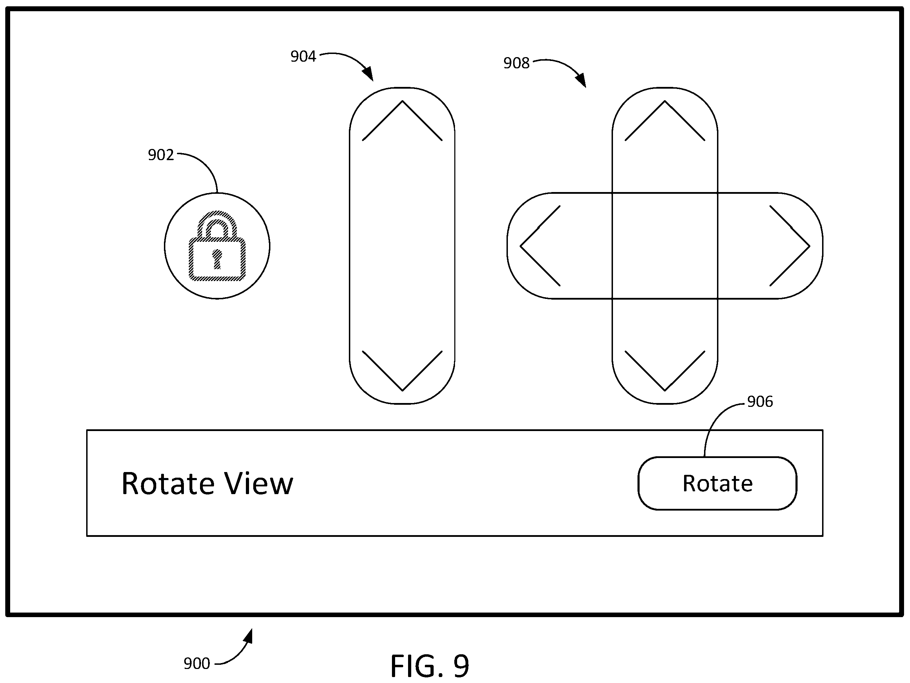

In some embodiments, control system 112 allows operator O to manually adjust virtual view 804 to account for the deviations, so that virtual view 804 can substantially match live camera view 802. The manual adjustments can include translation of virtual view 804 (e.g., advancement, retreat, up or down or left or right movement, etc.) to catch virtual view 804 back up to live camera view 802, without any effect on the elongate device. The translation can be a free translation and/or translation along planned path 806. For example, operator O can manually input an incremental (e.g., millimeter-by-millimeter or by another incremental unit) advancement or retreat along planned path 806 to bring virtual view 804 translationally closer to live camera view 802. As another example, operator O can manually input an incremental (e.g., millimeter-by-millimeter or by another incremental unit) translation along any translational degree of freedom to bring virtual view 804 translationally closer to live camera view 802. The manual adjustments can also include manual rotation of virtual view 804, without any effect on the elongate device. For example, operator O can activate a rotation mode, in which operator O can manually rotate virtual view 804 along any rotational degree of freedom using a control device (e.g., a trackball, a mouse, a touch-sensitive display on which virtual view 804 is displayed) to rotate virtual view 804 to more closely match live camera view 802. The manual adjustments might not affect the linking status; operator O can continue to move the elongate device using another control device or control modality. In some embodiments, control system 112 can also update the current registration based on the manual adjustments. For example, operator O could command control system 112 to map live camera view 802 and/or the current pose of the elongate device to virtual view 804 as manually adjusted, and control system 112 could update the registration based on that mapping. A graphical user interface associated with these manual adjustments is described below with reference to FIG. 9.

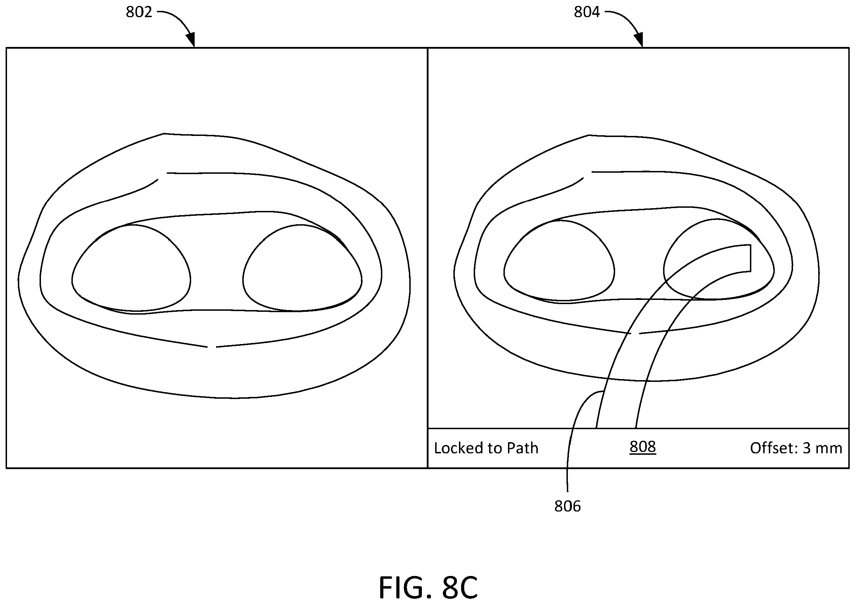

FIG. 8C illustrates live camera view 802 and virtual view 804 at a third time, in particular after manual adjustments to virtual view 804 as shown in FIG. 8B. As shown in FIG. 8C and compared to FIG. 8B, the branching passageway in virtual view 804 appears closer and better matches translationally the branching passageway as shown in live camera view 802. Additionally, virtual view 804 has been rotated to better match live camera view 802. After the manual adjustments to virtual view 804, operator O may continue to advance the elongate device using, for example, live camera view 802, and the images displayed in virtual view 804 may continue to be locked to planned path 806. As the elongate device is advanced, virtual view 804 might not appropriately match live camera view 802 at a future time, and the operator O may again manually rotate and/or translate virtual view 804 to more closely match live camera view 802.

While FIGs. 8A-8C omit navigation indicators (e.g., navigation indicators 506/606) and an orientation guide (e.g., orientation guide 508/608), in various embodiments navigation indicators and an orientation guide can be displayed concurrently with live camera view 802 and/or virtual view 804 in a manner similar to that described above with reference to FIGs. 5-6. Further, the navigation indicators and/or orientation guide can be updated based on the movement of the elongate device, movement along planned path 806, and/or manual adjustments to virtual view 804 in the linking mode.

Returning to information bar 808, an offset value indicates a difference between a position in the patient anatomy as shown in live camera view 802 and a position in the 3D model of the patient anatomy as shown in virtual view 804. The difference can indicate a degree of mismatch between live camera view 802 and virtual view 804. In some embodiments, the control system 112 can determine the offset value and/or otherwise determine that live camera view 802 and virtual view 804 do not match (e.g., do not match above a predefined tolerance) by comparing the current pose of the elongate device within the patient anatomy and portions of the patient anatomy in proximity of the current pose of the elongate device to the 3D model of the patient anatomy according to the current registration. Control system 112 can also, additionally or alternatively, determine the offset value by determining a distance between the elongate device in the current pose to the target location in the patient anatomy, determining a distance between the current position on planned path 806 to the target location in the 3D model of the patient anatomy, and comparing the two determined distances. The offset value can be expressed as a distance value indicating a distance between the pose of the elongate device and a current position in the 3D model of the patient anatomy as shown in virtual view 804. For example, in FIG. 8A an offset of 0 mm is displayed in information bar 808. In FIG. 8B, after some deviations, an offset of 20 mm is displayed in information bar 808. In FIG. 8C, after manual adjustments, an offset of 3 mm is displayed in information bar 808. An example of determining a match or non-match between a live camera view and a virtual view is described in PCT Publication WO 2019/245818 (published December 26, 2019) (disclosing "Systems and Methods Related to Registration for Image Guided Surgery"), which is incorporated by reference herein in its entirety.

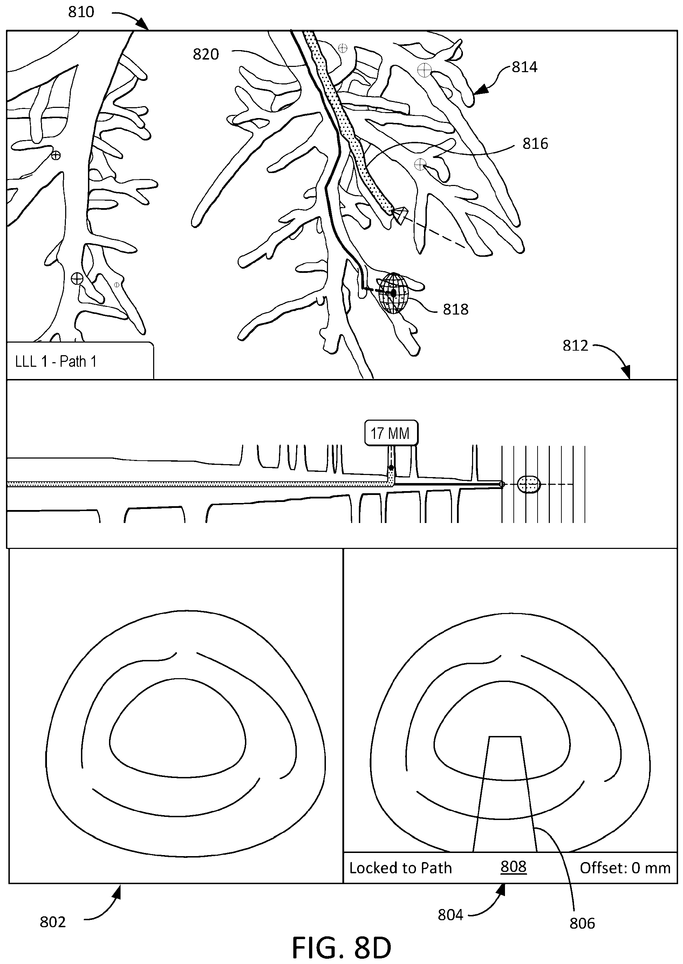

Additional user interfaces are possible where in addition to or instead of virtual view 804, one or more other views generated from the 3D model of the patient anatomy and the current registration between the 3D model and the patient anatomy are also provided. FIG. 8D is a simplified diagram of a user interface having a live camera view, a virtual view, a tree view, and a navigational view, according to some embodiments. And although FIG. 8D is shown with the virtual view, the tree view, and the navigational view, one or more of these views can be omitted from the user interface. Similar to the user interfaces of FIGs. 8A-8C, FIG. 8D includes live camera view 802, virtual view 804, planned path 806, and information bar 808. FIG. 8D also includes examples of a tree view 810 and a navigation view 812. Live camera view 802, virtual view 804, tree view 810, and navigation view 812 can be displayed as part of graphical user interface 400 while operating in a navigation mode. Tree view 810 includes a rendering 814 of the 3D model with a depiction 816 of the elongate device where the elongate device is located within the passageways of the patient anatomy of the 3D model. Tree view 810 further includes a target location 818, such as a target location within the passageways to which the elongate device is to be navigated. Tree view 810 also includes a planned path 820 showing the path through the passageways that the elongate device can use to reach target location 818. Planned path 820 corresponds to the tree view version of planned path 806. Navigation view 812 depicts widths and branching relationships of various anatomical passages along the length of navigation path 820, as well as the progress of the elongate device along navigation path 820.

FIG. 9 is a simplified diagram of a graphical user interface for manually adjusting a virtual view, according to some embodiments. Graphical user interface 900 can be displayed as a part of graphical user interface 400. Graphical user interface 900 can be displayed concurrently with live camera view 802 and/or virtual view 804 on the same display or on a different display within display system 110. Additionally or alternatively, graphical user interface 900 can be displayed as, for example, a pop-up view (e.g., operator O brings up graphical user interface 900 as a pop-up from a menu) or a switched view in place of any other view in graphical user interface 400 (e.g., operator O switches from local view 430 to graphical user interface 900).

Graphical user interface 900 includes one or more user interface elements associated the linking mode and/or manual adjustments of virtual view 804. As shown, graphical user interface 900 includes a linking status toggle button 902 to toggle the status of a linking mode with respect to the elongate device and virtual view 804. Graphical user interface 900 also includes up/down arrow button 904 for inputting a manual incremental adjustment of virtual view 804 along a first translational degree of freedom or along planned path 806 (e.g., advancement forward or retreat backward) without affecting the elongate device. For example, operator O could activate the up-arrow on button 904 to manually advance virtual view 804 without moving the elongate device and activate the down-arrow on button 904 to manually retreat virtual view 804 without affecting the elongate device. Graphical user interface 900 can also include arrow buttons 908 for inputting similar manual incremental adjustments of virtual view 804 along one or more additional translational degrees of freedom. For example, arrow buttons 908 as shown includes up/down arrows for inputting adjustments of virtual view 804 up or down, and left/right arrows for inputting adjustments of virtual view 804 to the left or to the right.

Graphical user interface 900 can further include a rotation mode toggle button 906 for toggling a rotation mode for manually rotating virtual view 804. While the rotation mode is active, operator O can manually rotate virtual view 804 using a control device, without moving or rotating or otherwise affecting the elongate device.

It should be appreciated that graphical user interface 900 is merely one example. Graphical user interface 900 can include more or fewer user interface elements than as shown, can be incorporated into or another view within graphical user interface 400, and/or the like.

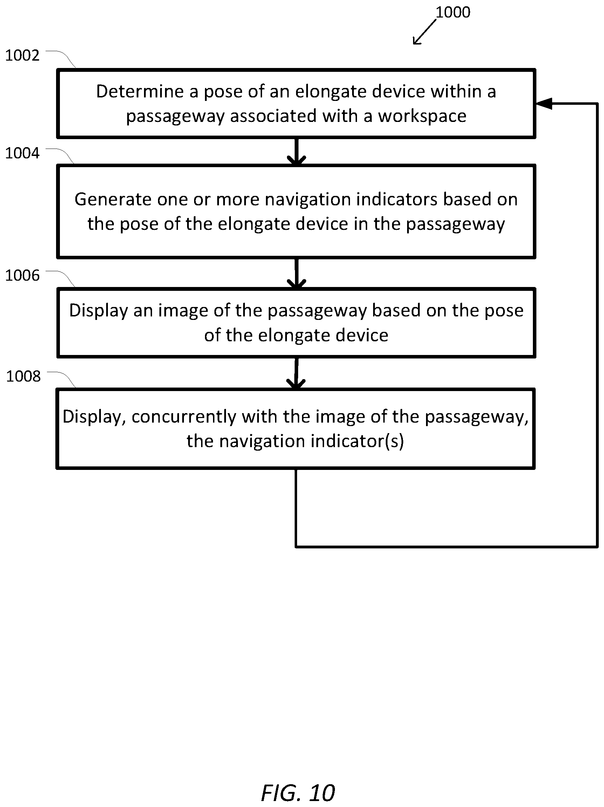

FIG. 10 is a flow chart of method steps for providing navigation guidance for an elongate device, according to some embodiments. Although the method steps are described with respect to the systems of FIGs. 1-9, persons skilled in the art will understand that any system configured to perform the method steps, in any order, falls within the scope of the various embodiments. In some embodiments, one or more of the steps 1002-1008 of method 1000 may be implemented, at least in part, in the form of executable code stored on one or more non-transient, tangible, machine readable media that when run by one or more processors (e.g., one or more processors of control system 112) would cause the one or more processors to perform one or more of the steps 1002-1008.

As shown, method 1000 begins at step 1002, where control system 112 determines a pose of an elongate device within a passageway associated with a workspace. In some embodiments, the elongate device can correspond to instrument 104, elongate device 202, a catheter, and/or the like. Control system 112 can determine a current pose (e.g., current position and/or orientation) of an elongate device within a passageway in the workspace (e.g., within a passageway in the patient anatomy of patient P). Control system 112 can determine the pose of the elongate device by, for example, obtaining data from sensor system 108.

At step 1004, control system 112 generates one or more navigation indicators based on the pose of the elongate device in the passageway. Control system 112 generates (or updates) navigation indicators (e.g., navigation indicators 506/606) and optionally an orientation guide (e.g., orientation guide 508/608) based on the current pose of the elongate device relative to the passageway and the workspace (e.g., which half of the patient anatomy relative to the sagittal plane is the elongate device located, orientation of the elongate device relative to the patient anatomy).

At step 1006, control system 112 displays an image of the passageway based on the pose of the elongate device. Control system 112 displays, on display system 110, a live camera view (e.g., live camera view 502/602) captured from a camera on or near a distal end of the elongate device, from the pose of the elongate device, and/or a virtual view (e.g., virtual view 504/604) associated with the pose of the elongate device and according to a current registration.

At step 1008, control system 112 displays, concurrently with the image of the passageway, the navigation indicators. Control system 112 displays, over the live camera view and/or the virtual view, the navigation indicators generated and/or updated in step 1004. Control system 112 can also display the orientation guide generated and/or updated in step 1004 concurrently and aside from the live camera view and/or the virtual view. Method 1000 can then return to step 1002 to determine a new current pose of the elongate device and proceed through method 1000 to update the image and/or the navigation indicators.

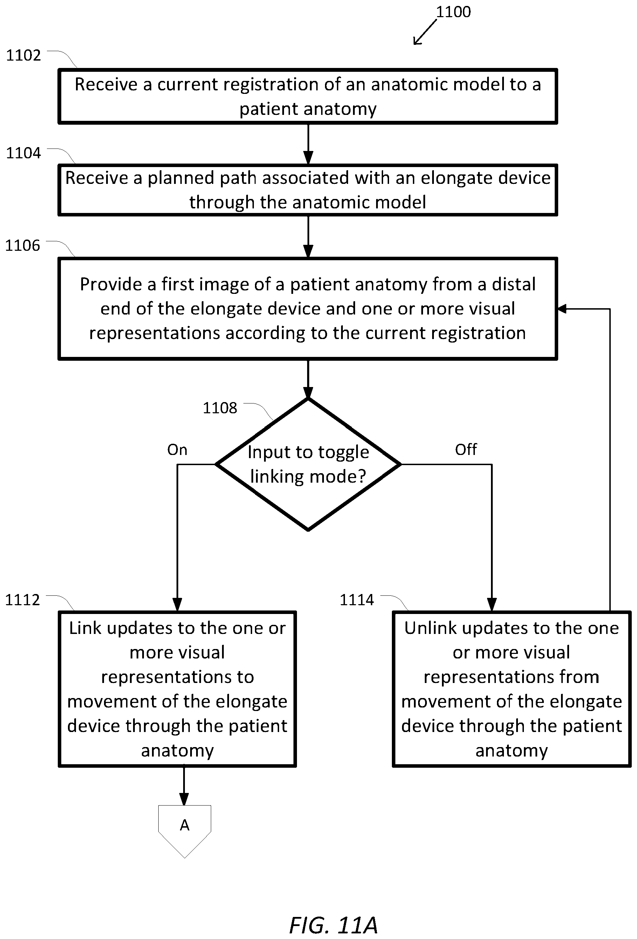

FIGs. 11A-11B is a flow chart of method steps for adjusting a virtual representation, according to some embodiments. Although the method steps are described with respect to the systems of FIGs. 1-9, persons skilled in the art will understand that any system configured to perform the method steps, in any order, falls within the scope of the various embodiments. In some embodiments, one or more of the steps 1102-1120 of method 1100 may be implemented, at least in part, in the form of executable code stored on one or more non-transient, tangible, machine readable media that when run by one or more processors (e.g., one or more processors of control system 112) would cause the one or more processors to perform one or more of the steps 1102-1120.

As shown, method 1100 begins at step 1102, where control system 112 receives a current registration of an anatomic model to the patent anatomy. In an example, the current registration can be the registration of a 3D model to a patient anatomy. The registration can be generated and/or updated before a procedure or during the procedure and received by control system 112.

At step 1104, control system 112 receives a planned path associated with an elongate device through the anatomic model. In some embodiments, the elongate device can correspond to instrument 104, elongate device 202, a catheter, and/or the like. Control system 112 can receive a saved plan that includes a planned path for an elongate device through the 3D model to one or more target locations (e.g., planned path 610,r 806, or 820).

At step 1106, control system 112 provides a first image of a patient anatomy from a distal end of the elongate device and one or more visual representations of the anatomic model. Control system 112 can display, on display system 110, a live camera view from the distal end of the elongate device in a current pose in the patient anatomy and one or more views associated with the current pose of the elongate device according to the current registration received in step 1102. Control system 112 can display the planned path received in step 1104 concurrently with the virtual view. For example, control system 112 can provide a live camera view (e.g., live camera view 502, 602, or 802) and one or more additional views, such as a virtual view (e.g., virtual view 504, 604, or 804), a tree view (e.g., tree view 810), and/or a navigation view (e.g., navigational view 812) and display those on display system 110. Control system 112 can further display a planned path (e.g., planned path 610, 806, or 820) in one or more of the additional views on display system 110.

At step 1108, control system 112 can receive an input toggling a linking mode between movement of the elongate device and the one or more visual representations (e.g., the one or more visual representations provided in step 1106). In some embodiments, updates to one or more of the additional views (e.g., virtual view 804, tree view 810, and/or navigation view 812) can be linked to movement of the elongate device. Control system 112 can receive the toggle input from operator O via toggle button 902. If the toggle input is an input to deactivate (toggle off) the linking mode, then method 1100 proceeds to step 1114, where control system 112 deactivates the linking mode, which includes delinking updates of the first visual representation from movement of the elongate device through the patient anatomy. Method 1100 can then proceed back to step 1106. While the linking mode is deactivated, the one or more visual representations (e.g., virtual view 804, tree view 810, and/or navigation view 812) are provided based on the current registration (e.g., the current pose of the elongate device is mapped to a pose in the 3D model of the patient anatomy based on the registration, and the one or more visual representations displayed according to the pose in the 3D model).

If the toggle input is an input to activate (toggle on) the linking mode, then method 1100 proceeds to step 1112, where control system 112 links updates to the one or more visual representations to movement of the elongate device through the patient anatomy. Control system 112 can link one or more visual representations and updates thereof to movement of the elongate device through the patient anatomy, so that control system 112 need not rely on the registration for updating the one or more visual representations. For example, as operator O inserts or retracts the elongate device via input on a control device (e.g., a scroll wheel or trackball), control system 112 could update virtual view 804 and/or a depicted position of the elongate device along the planned path in one or more of tree view 810 and/or navigation view 812 by an amount corresponding to the control device input as opposed to determining the current pose of the elongate device and mapping that pose to the 3D model based on the registration.

At step 1116, based on a movement of the elongate device, control system 112 may provide a second image of the patient anatomy and one or more visual representations of the anatomic model according to the linking. While the linking mode is active, operator O can make inputs via a control device (e.g., a scroll wheel or trackball) to move the elongate device with respect to the patient anatomy. Based on the movement of the elongate device via the control device and the active linking mode, control system 112 can update one or more of virtual view 804, tree view 810, and/or navigation view 812 and live camera view 802. Control system 112 can update live camera view 802 with new images captured from the elongate device. Control system 112 can update one or more of virtual view 804 and/or the depiction of the position of the elongate device along the planned path tree view 810, and/or navigation view 812 by an amount along planned path corresponding to the movement of the elongate device as input via the control device.

At step 1118, control system 112 receives one or more second inputs corresponding to adjustments to the one or more visual representations. Control system 112 can receive one or more inputs, from a control device, corresponding to manual adjustments of virtual view 804 by operator O. The manual adjustments can include translation of virtual view 804 along one or more translational degrees of freedom, advance or retreat along planned path, and/or rotation of virtual view 804 along one or more rotational degrees of freedom. The manual adjustments can also include advancing or retreating the depiction of the elongate device along the planned path in tree view 810 and/or navigation view 812. For example, operator O could interact with arrow buttons 904 and/or 908 to translate virtual view 804 without causing movement of the elongate device. As another example, operator O could interact with rotation mode toggle button 906 to activate a rotation mode for virtual view 804 and then rotate virtual view 804 via a control device while the rotation mode is active without causing movement of the elongate device. In some embodiments, control system 112 can determine that live camera view 802 has deviated from virtual view 804 (e.g., live camera view 802 and virtual view 804 do not match). Control system 112 can determine an offset value to represent the difference between live camera view 802 and virtual view 804. Control system 112 can display that offset value in information bar 808 along with virtual view 804 or provide any other indication of a mismatch. Operator O can make the manual adjustment inputs in response to the offset value or indication of mismatch or on his own initiative (e.g., control system 112 might not determine an offset or provide an indication of mismatch, and operator O can identify a mismatch from visual inspection of live camera view 802 and virtual view 804).

At step 1120, based on the second inputs, control system 112 adjusts the one or more visual representations. In response to the manual adjustment inputs, control system 112 adjusts the one or more visual representations and displays the adjusted one or more visual representations. In some embodiments, control system 112 can also update the registration based on the manual adjustments to virtual view 804. For example, operator O could command control system 112 to map live camera view 802 and/or the current pose of the elongate device to virtual view 804 as manually adjusted, and control system 112 would update the registration based on that mapping. In some embodiments, from step 1120, method 1100 can loop back to perform one or more steps of method 1100 again. For example, method 1100 could, from step 1120, return to step 1116 to further update virtual view 804 based on further movement of the elongate device according to an active linking mode. As another example, method 1100 could, from step 1120, return to step 1108 to determine whether the linking mode has been turned off.

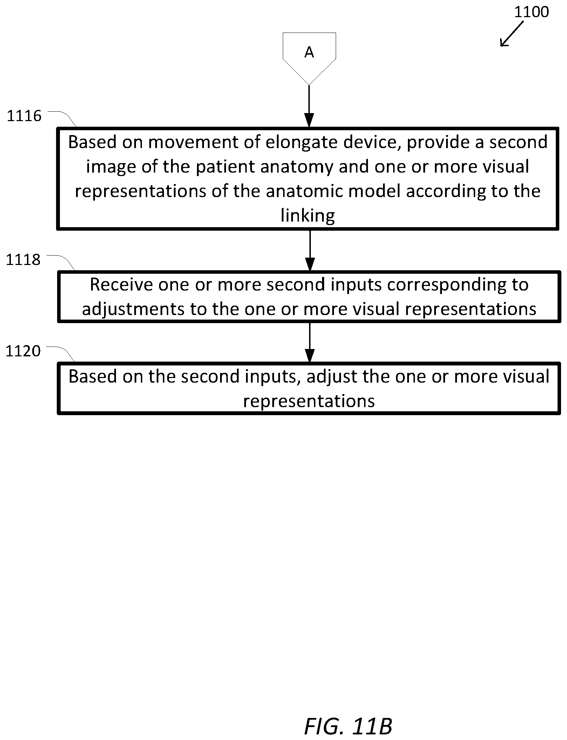

As discussed above and further emphasized here, Figures 11A and 11B are merely examples, which should not unduly limit the scope of the claims. Additional variations, alternatives, and modifications are possible. In some embodiments, one or more additional changes to one or more visual characteristics of the one or more visual representations are made to assist the user in determining whether the one or more virtual representations in the user interface are being generated using the linking mode (e.g., based on the commanded motion of the elongate device) or not (e.g., based on the current registration). In some examples, the one or more additional changes to the one or more visual characteristics include changing one or more labels on the user interface to identify the type of navigation mode, such as using the "locked to path" label as depicted in FIGs. 8A-8D to indicate that the current navigation mode is the linking mode and other suitable text indicating when the navigation mode is not in the linking mode. In some examples, one or more colors of the elements (e.g., any of the border, background, anatomical features, planned path, and/or the target) in virtual view 804, tree view 810, and/or navigation view 812 can be different depending upon whether the one or more visual representations are being displayed by process 1106 (not in linking mode) or processes 1116 and/or 1120 (in linking mode). For example, a first color scheme can be used during process 1106 and a second color scheme with one or more colors that differ from the first color scheme can be used during processes 1116 and/or 1120 (in linking mode). In some examples, one or more of a mesh, a transparency, a symbol, an icon, and/or any other visual characteristic of the elements in virtual view 804, tree view 810, and/or navigation view 812 can be different depending upon whether the one or more visual representations are being displayed by process 1106 or processes 1116 and/or 1120.

In sum, various techniques provide navigational assistance for an elongate device during an image-guided procedure. In a first technique, a computing system determines a pose of an elongate device (e.g., an instrument, such as a catheter) within a passageway in a workspace (e.g., a patient anatomy). The computing system generates one or more navigation indicators (e.g., indicators of anatomical directions) based on the pose of the elongate device. The computing system displays concurrently an image corresponding to a view associated with the pose of the elongate device within the passageway and one or more of the navigation indicators. The navigation indicators can indicate anatomical directions, directions in a coordinate frame, or compass directions. The computing system can also display an orientation guide that provides information indicating the current pose of the elongate device from a perspective external to the workspace. In a second technique, while updates to a virtual view are linked to movement of an elongate device, the computing system can receive inputs for manually adjusting the virtual view based on the inputs, without affecting the elongate device.

At least one advantage and technical improvement of the disclosed techniques relative to the prior art is that, with the disclosed techniques, navigational assistance for an elongate device is provided in a clearer and more informative manner to an operator relative to conventional approaches. The navigational assistance can be used with live image-based guidance and/or dynamic registration guidance. Accordingly, an operator of the elongate device can more effectively navigate the elongate device within one or more passageways (e.g., within a patient) with reduced likelihood of navigating down an undesired path and undesired backtracking. Another advantage and technical improvement is that, while changes to the pose of an elongate device (e.g., movement of the elongate device) is linked to movement along a planned path in dynamic registration guidance, a virtual view on the planned path can be adjusted to better match a live view from the elongate device without causing a change in the pose of the elongate device. Accordingly, an operator can manually adjust a virtual view in dynamic registration guidance when the virtual view does not match the live view. These technical advantages provide one or more technological advancements over prior art approaches.
1. In some embodiments, a method comprises determining a pose of an elongate device within a passageway and based on the pose of the elongate device, causing to be displayed on a display system an image of the passageway and one or more navigation indicators associated with one or more directions within a workspace containing the passageway, wherein the one or more navigation indicators are displayed over the image of the passageway.
2. The method according to clause 1, wherein the elongate device comprises a catheter.
3. The method according to clause 1 or clause 2, wherein the pose of the elongate device comprises a position of the elongate device within the workspace and an orientation of the elongate device relative to the workspace.
4. The method according to any of clauses 1-3, wherein the one or more navigation indicators comprise an indicator of a direction within the workspace relative to a distal end of the elongate device.
5. The method according to any of clauses 1-4, wherein the direction within the workspace comprises an anterior direction, a posterior direction, a lateral direction, a medial direction, a superior direction, or an inferior direction.
6. The method according to any of clauses 1-5, wherein the direction within the workspace comprises a direction along an axis in a Cartesian coordinate frame.
7. The method according to any of clauses 1-6, wherein the direction within the workspace comprises a cardinal direction.
8. The method according to any of clauses 1-7, further comprising causing to be displayed on the display system an orientation guide comprising a three-dimensional (3D) representation of the workspace and a plurality of second navigation indicators based on the pose of the elongate device relative to the workspace.
9. The method according to any of clauses 1-8, wherein the orientation guide comprises a virtual sphere, and the 3D representation of the workspace is centered within the virtual sphere.
10. The method according to any of clauses 1-9, wherein the orientation guide comprises one or more equator lines along a virtual surface of the virtual sphere.
11. The method according to any of clauses 1-10, wherein a first equator line included in the one or more equator lines includes a plurality of gaps placed at regular intervals along the first equator line.
12. The method according to any of clauses 1-11, wherein each of the equator lines correspond to an anatomical plane.
13. The method according to any of clauses 1-12, wherein the orientation guide further comprises one or more indicators of one or more planes in a coordinate frame associated with the orientation guide.
14. The method according to any of clauses 1-13, further comprising highlighting a first portion of the 3D representation of the workspace based on a position of the elongate device relative to the workspace.
15. The method according to any of clauses 1-14, further comprising determining a change in the pose of the elongate device, wherein the change in the pose includes a change in a position of the elongate device across a plane bisecting the workspace; and based on the change in the pose of the elongate device: highlighting a second portion of the 3D representation and un-highlighting the first portion of the 3D representation.
16. The method according to any of clauses 1-15, wherein the plane bisecting the workspace comprises a sagittal plane of the workspace, the first portion of the 3D representation corresponds to a first side of the sagittal plane, and the second portion of the 3D representation corresponds to an opposite side of the sagittal plane from the first side.
17. The method according to any of clauses 1-16, further comprising, based on the change in the pose of the elongate device, switching positions of a first one and a second one of the plurality of second navigation indicators included in the orientation guide.
18. The method according to any of clauses 1-17, wherein: the first one of the plurality of second navigation indicators included in the orientation guide comprises a medial direction indicator and the second one of the plurality of second navigation indicators included in the orientation guide comprises a lateral direction indicator.
19. The method according to any of clauses 1-18, further comprising: determining a change in the pose of the elongate device, wherein the change in the pose includes a change in a position of the elongate device across a plane bisecting the workspace; and based on the change in the pose of the elongate device, switching positions of a first one and a second one of the one or more navigation indicators.
20. The method according to any of clauses 1-19, wherein the first one of the one or more navigation indicators comprises a medial direction indicator and the second one of the one or more navigation indicators comprises a lateral direction indicator.
21. The method according to any of clauses 1-20, wherein the workspace comprises an interior anatomy of a patient.
22. The method according to any of clauses 1-21, wherein the image of the passageway comprises a virtual representation of the passageway.
23. The method according to any of clauses 1-22, wherein the image of the passageway comprises an image of the passageway captured by an imaging device associated with the elongate device.
24. The method according to any of clauses 1-23, further comprising: causing to be displayed, on the display system, a virtual representation of a path through the passageway based on the pose of the elongate device; receiving a first input via a first input device; in response to the first input, causing to be displayed a second image of the passageway corresponding to movement of the elongate device; receiving a second input via a second input device; and in response to the second input, causing to be displayed a third image of the passageway corresponding to a change to the second image along at least one of a translational or a rotational degree of freedom.
25. In some embodiments,, a method comprises: causing to be displayed on a display system a first virtual representation of a passageway based on a pose of an elongate device and a virtual representation of a path associated with the elongate device; receiving a first input via a first control device; in response to the first input while the elongate device is being navigated in a linking mode, causing to be displayed on the display system a second virtual representation of the passageway corresponding to movement along the virtual representation of the path, wherein the movement along the virtual representation of the path is based on the first input; receiving a second input via a second control device; and in response to the second input, causing to be displayed on the display system a third virtual representation of the passageway corresponding to a change to the second virtual representation along at least one of a translational or rotational degree of freedom, wherein the change to the second virtual representation is based on the second input.
26. The method according to clause 25, further comprising in response to the first input, causing a change in the pose of the elongate device and in response to the second input, maintaining the pose of the elongate device.
27. The method according to clause 25 or clause 26, wherein the second input comprises a translation of the second virtual representation along a first translational degree of freedom.
28. The method according to any of clauses 25-27, wherein the second input comprises a rotation of the second virtual representation along a first rotational degree of freedom.
29. The method according to any of clauses 25-28, further comprising determining an offset between a live image of the passageway and the second virtual representation of the passageway.
30. The method according to any of clauses 25-29, further comprising updating a live image of the passageway based on a change in the pose of the elongate device.
31. The method according to any of clauses 25-30, further comprising receiving a command from an operator to deactivate the linking mode and in response to the command, causing to display on the display system a fourth virtual representation of the passageway corresponding to movement along the virtual representation of the path, wherein the movement along the virtual representation of the path is based on a registration between the elongate device and a model of the passageway.
32. The method according to any of clauses 25-31, wherein one or more first visual characteristics of a first element depicted in the third virtual representation is different from one or more corresponding second visual characteristics of the first element depicted in the fourth virtual representation.
33. The method according to any of clauses 25-32, wherein one or more first visual characteristics comprise a first color for the first element depicted and the one or more second visual characteristics comprise a second color for the first element, the second color being different from the first color.
34. The method according to any of clauses 25-33, wherein the one or more first visual characteristics include one or more of a mesh, a transparency, a symbol, or an icon for the first element.
35. The method according to any of clauses 25-34, wherein the first element is the path, the passageway, a target, a border, or a background.
36. The method according to any of clauses 25-35, further comprising displaying a label indicating whether the linking mode is activated or deactivated.
37. **In** some embodiments, one or more non-transitory machine-readable media comprising a plurality of machine-readable instructions which when executed by one or more processors are adapted to cause the one or more processors to perform the method of any one of clauses 1-36.Any and all combinations of any of the claim elements recited in any of the claims and/or any elements described in this application, in any fashion, fall within the contemplated scope of the present disclosure and protection.

The descriptions of the various embodiments have been presented for purposes of illustration, but are not intended to be exhaustive or limited to the embodiments disclosed. Many modifications and variations will be apparent to those of ordinary skill in the art without departing from the scope and spirit of the described embodiments.

Aspects of the present embodiments may be embodied as a system, method or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as a "module," a "system," or a "computer." **In** addition, any hardware and/or software technique, process, function, component, engine, module, or system described in the present disclosure may be implemented as a circuit or set of circuits. Furthermore, aspects of the present disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

Aspects of the present disclosure are described above with reference to flowchart illustrations and/or block diagrams of methods, apparatus (systems) and computer program products according to embodiments of the disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine. The instructions, when executed via the processor of the computer or other programmable data processing apparatus, enable the implementation of the functions/acts specified in the flowchart and/or block diagram block or blocks. Such processors may be, without limitation, general purpose processors, special-purpose processors, application-specific processors, or field-programmable gate arrays.

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

While the preceding is directed to embodiments of the present disclosure, other and further embodiments of the disclosure may be devised without departing from the basic scope thereof, and the scope thereof is determined by the claims that follow.

### Preferred Embodiments

The 55 claims of the parent application are reproduced immediately below as clauses. These clauses define preferred embodiments. The applicant reserves the right to pursue protection for the combinations of features set out in these clauses, and/or for any other subject matter contained in the parent application as filed, either in the present divisional application or in a further application divided from the present divisional application.
1. A system, comprising:
   an elongate device;
   a display system;
   one or more processors; and
   memory storing instructions that, when executed by the one or more processors, cause the one or more processors to:
      determine a pose of the elongate device within a passageway; and
      based on the pose of the elongate device, display on the display system:
         an image of the passageway, and
         one or more navigation indicators associated with one or more directions within a workspace containing the passageway, wherein the one or more navigation indicators are displayed over the image of the passageway.
2. The system of clause 1, wherein the elongate device comprises a catheter.
3. The system of clause 1, wherein the pose of the elongate device comprises a position of the elongate device within the workspace and an orientation of the elongate device relative to the workspace.
4. The system of clause 1, wherein the one or more navigation indicators comprise an indicator of a direction within the workspace relative to a distal end of the elongate device.
5. The system of clause 4, wherein the direction within the workspace comprises an anterior direction, a posterior direction, a lateral direction, a medial direction, a superior direction, or an inferior direction.
6. The system of clause 4, wherein the direction within the workspace comprises a direction along an axis in a Cartesian coordinate frame.
7. The system of clause 4, wherein the direction within the workspace comprises a cardinal direction.
8. The system of clause 1, wherein the memory stores instructions that, when executed by the one or more processors, cause the one or more processors to display on the display system an orientation guide comprising:
   a three-dimensional (3D) representation of the workspace; and
   a plurality of second navigation indicators based on the pose of the elongate device relative to the workspace.
9. The system of clause 8, wherein the orientation guide comprises a virtual sphere, and the 3D representation of the workspace is centered within the virtual sphere.
10. The system of clause 9, wherein the orientation guide comprises one or more equator lines along a virtual surface of the virtual sphere.
11. The system of clause 10, wherein a first equator line included in the one or more equator lines includes a plurality of gaps placed at regular intervals along the first equator line.
12. The system of clause 10, wherein each of the equator lines correspond to an anatomical plane.
13. The system of clause 8, wherein the orientation guide further comprises one or more indicators of one or more planes in a coordinate frame associated with the orientation guide.
14. The system of clause 8, wherein the memory stores instructions that, when executed by the one or more processors, cause the one or more processors to highlight a first portion of the 3D representation of the workspace based on a position of the elongate device relative to the workspace.
15. The system of clause 14, wherein the memory stores instructions that, when executed by the one or more processors, cause the one or more processors to:
   determine a change in the pose of the elongate device, wherein the change in the pose includes a change in a position of the elongate device across a plane bisecting the workspace; and
   based on the change in the pose of the elongate device:
      highlight a second portion of the 3D representation, and
      un-highlight the first portion of the 3D representation.
16. The system of clause 15, wherein:
   the plane bisecting the workspace comprises a sagittal plane of the workspace,
   the first portion of the 3D representation corresponds to a first side of the sagittal plane, and
   the second portion of the 3D representation corresponds to an opposite side of the sagittal plane from the first side.
17. The system of clause 15, wherein the memory stores instructions that, when executed by the one or more processors, cause the one or more processors to, based on the change in the pose of the elongate device, switch positions of a first one and a second one of the plurality of second navigation indicators included in the orientation guide.
18. The system of clause 17, wherein:
   the first one of the plurality of second navigation indicators included in the orientation guide comprises a medial direction indicator, and
   the second one of the plurality of second navigation indicators included in the orientation guide comprises a lateral direction indicator.
19. The system of clause 1, wherein the memory stores instructions that, when executed by the one or more processors, cause the one or more processors to:
   determine a change in the pose of the elongate device, wherein the change in the pose includes a change in a position of the elongate device across a plane bisecting the workspace; and
   based on the change in the pose of the elongate device, switch positions of a first one and a second one of the one or more navigation indicators.
20. The system of clause 19, wherein:
   the first one of the one or more navigation indicators comprises a medial direction indicator, and
   the second one of the one or more navigation indicators comprises a lateral direction indicator.
21. The system of any one of clauses 1-20, wherein the workspace comprises an interior anatomy of a patient.
22. The system of any one of clauses 1-20, wherein the image of the passageway comprises a virtual representation of the passageway.
23. The system of any one of clauses 1-20, wherein the image of the passageway comprises an image of the passageway captured by an imaging device associated with the elongate device.
24. The system of any one of clauses 1-20, further comprising:
   a first input device, and
   a second input device; and
   wherein the memory stores instructions that, when executed by the one or more processors, cause the one or more processors to:
      display, on the display system, a virtual representation of a path through the passageway based on the pose of the elongate device;
      receive a first input via the first input device;
      in response to the first input, display a second image of the passageway corresponding to movement of the elongate device;
      receive a second input via the second input device; and
      in response to the second input, display a third image of the passageway corresponding to a change to the second image along at least one of a translational or a rotational degree of freedom.
25. An apparatus, comprising:
   one or more processors; and
   memory storing instructions that, when executed by the one or more processors, cause the one or more processors to:
      determine a pose of an elongate device within a passageway; and
      based on the pose of the elongate device, cause to display on a display system:
         an image of the passageway, and
         one or more navigation indicators associated with one or more directions within a workspace containing the passageway, wherein the one or more navigation indicators are displayed over the image of the passageway.
26. The apparatus of clause 25, wherein the elongate device comprises a catheter.
27. The apparatus of clause 25, wherein the pose of the elongate device comprises a position of the elongate device within the workspace and an orientation of the elongate device relative to the workspace.
28. The apparatus of clause 25, wherein the one or more navigation indicators comprise an indicator of a direction within the workspace relative to a distal end of the elongate device.
29. The apparatus of clause 28, wherein the direction within the workspace comprises an anterior direction, a posterior direction, a lateral direction, a medial direction, a superior direction, an inferior direction, an axis in a Cartesian coordinate frame, or a cardinal direction.
30. The apparatus of clause 25, wherein the memory stores instructions that, when executed by the one or more processors, cause the one or more processors to display on the display system an orientation guide comprising:
   a three-dimensional (3D) representation of the workspace; and
   a plurality of second navigation indicators based on the pose of the elongate device relative to the workspace.
31. The apparatus of clause 30, wherein the orientation guide comprises a virtual sphere, and the 3D representation of the workspace is centered within the virtual sphere.
32. The apparatus of clause 31, wherein the orientation guide comprises one or more equator lines along a virtual surface of the virtual sphere.
33. The apparatus of clause 32, wherein each of the equator lines correspond to an anatomical plane.
34. The apparatus of clause 30, wherein the orientation guide further comprises one or more indicators of one or more planes in a coordinate frame associated with the orientation guide.
35. The apparatus of clause 30, wherein the memory stores instructions that, when executed by the one or more processors, cause the one or more processors to highlight a first portion of the 3D representation of the workspace based on a position of the elongate device relative to the workspace.
36. The apparatus of clause 35, wherein the memory stores instructions that, when executed by the one or more processors, cause the one or more processors to:
   determine a change in the pose of the elongate device, wherein the change in the pose includes a change in a position of the elongate device across a plane bisecting the workspace; and
   based on the change in the pose of the elongate device:
      highlight a second portion of the 3D representation, and
      un-highlight the first portion of the 3D representation.
37. The apparatus of clause 36, wherein:
   the plane bisecting the workspace comprises a sagittal plane of the workspace,
   the first portion of the 3D representation corresponds to a first side of the sagittal plane, and
   the second portion of the 3D representation corresponds to an opposite side of the sagittal plane from the first side.
38. The apparatus of clause 36, wherein the memory stores instructions that, when executed by the one or more processors, cause the one or more processors to, based on the change in the pose of the elongate device, switch positions of a first one and a second one of the plurality of second navigation indicators included in the orientation guide.
39. The apparatus of clause 38, wherein:
   the first one of the plurality of second navigation indicators included in the orientation guide comprises a medial direction indicator, and
   the second one of the plurality of second navigation indicators included in the orientation guide comprises a lateral direction indicator.
40. The apparatus of any one of clauses 25-39, wherein the workspace comprises an interior anatomy of a patient.
41. The apparatus of any one of clauses 25-39, wherein the image of the passageway comprises a virtual representation of the passageway.
42. The apparatus of any one of clauses 25-39, wherein the image of the passageway comprises an image of the passageway captured by an imaging device associated with the elongate device.
43. The apparatus of any one of clauses 25-39, wherein the memory stores instructions that, when executed by the one or more processors, cause the one or more processors to:
   display, on the display system, a virtual representation of a path through the passageway based on the pose of the elongate device;
   receive a first input via a first input device;
   in response to the first input, display a second image of the passageway corresponding to movement of the elongate device;
   receive a second input via a second input device; and
   in response to the second input, display a third image of the passageway corresponding to a change to the second image along at least one of a translational or a rotational degree of freedom.
44. A system, comprising:
   one or more processors; and
   memory storing instructions that, when executed by the one or more processors, cause the one or more processors to:
      cause to display on a display system:
         a first virtual representation of a passageway based on a pose of an elongate device, and
         a virtual representation of a path associated with the elongate device;
      receive a first input via a first control device;
      in response to the first input while the elongate device is being navigated in a linking mode, cause to display on the display system:
         a second virtual representation of the passageway corresponding to movement along the virtual representation of the path, wherein the movement along the virtual representation of the path is based on the first input;
      receive a second input via a second control device; and
      in response to the second input, cause to display on the display system:
         a third virtual representation of the passageway corresponding to a change to the second virtual representation along at least one of a translational or rotational degree of freedom, wherein the change to the second virtual representation is based on the second input.
45. The system of clause 44, wherein the memory stores instructions that, when executed by the one or more processors, cause the one or more processors to:
   in response to the first input, cause a change in the pose of the elongate device; and
   in response to the second input, maintain the pose of the elongate device.
46. The system of clause 44, wherein the second input comprises a translation of the second virtual representation along a first translational degree of freedom.
47. The system of clause 44, wherein the second input comprises a rotation of the second virtual representation along a first rotational degree of freedom.
48. The system of any one of clauses 44-47, wherein the memory stores instructions that, when executed by the one or more processors, cause the one or more processors to determine an offset between a live image of the passageway and the second virtual representation of the passageway.
49. The system of any one of clauses 44-47, wherein the memory stores instructions that, when executed by the one or more processors, cause the one or more processors to update a live image of the passageway based on a change in the pose of the elongate device.
50. The system of any one of clauses 44-47, wherein the memory stores instructions that, when executed by the one or more processors, cause the one or more processors to:
   receive a command from an operator to deactivate the linking mode; and
   in response to the command, cause to display on the display system a fourth virtual representation of the passageway corresponding to movement along the virtual representation of the path, wherein the movement along the virtual representation of the path is based on a registration between the elongate device and a model of the passageway.
51. The system of clause 50, wherein one or more first visual characteristics of a first element depicted in the third virtual representation is different from one or more corresponding second visual characteristics of the first element depicted in the fourth virtual representation.
52. The system of clause 51, wherein one or more first visual characteristics comprise a first color for the first element depicted and the one or more second visual characteristics comprise a second color for the first element, the second color being different from the first color.
53. The system of clause 51, wherein the one or more first visual characteristics include one or more of a mesh, a transparency, a symbol, or an icon for the first element.
54. The system of clause 51, wherein the first element is the path, the passageway, a target, a border, or a background.
55. The system of clause 50, wherein the memory stores instructions that, when executed by the one or more processors, cause the one or more processors to display a label indicating whether the linking mode is activated or deactivated.

## Claims

1. A system, comprising:
one or more processors; and
memory storing instructions that, when executed by the one or more processors, cause the one or more processors to:
cause to display on a display system:
a first virtual representation of a passageway based on a pose of an elongate device, and
a virtual representation of a path associated with the elongate device;
receive a first input via a first control device;
in response to the first input while the elongate device is being navigated in a linking mode, cause to display on the display system:
a second virtual representation of the passageway corresponding to movement along the virtual representation of the path, wherein the movement along the virtual representation of the path is based on the first input;
receive a second input via a second control device; and
in response to the second input, cause to display on the display system:
a third virtual representation of the passageway corresponding to a change to the second virtual representation along at least one of a translational or rotational degree of freedom, wherein the change to the second virtual representation is based on the second input.

2. The system of claim 1, wherein the memory stores instructions that, when executed by the one or more processors, cause the one or more processors to:
in response to the first input, cause a change in the pose of the elongate device; and
in response to the second input, maintain the pose of the elongate device.

3. The system of claim 1, wherein the second input comprises a translation of the second virtual representation along a first translational degree of freedom.

4. The system of claim 1, wherein the second input comprises a rotation of the second virtual representation along a first rotational degree of freedom.

5. The system of any one of claims 1-4, wherein the memory stores instructions that, when executed by the one or more processors, cause the one or more processors to determine an offset between a live image of the passageway and the second virtual representation of the passageway.

6. The system of any one of claims 1-4, wherein the memory stores instructions that, when executed by the one or more processors, cause the one or more processors to update a live image of the passageway based on a change in the pose of the elongate device.

7. The system of claim 6, wherein the live image of the passageway is received from an imaging device located at a distal end of the elongate device.

8. The system of any one of claims 1-4, wherein the memory stores instructions that, when executed by the one or more processors, cause the one or more processors to:
receive a command from an operator to deactivate the linking mode; and
in response to the command, cause to display on the display system a fourth virtual representation of the passageway corresponding to movement along the virtual representation of the path, wherein the movement along the virtual representation of the path is based on a registration between the elongate device and a model of the passageway.

9. The system of claim 8, wherein one or more first visual characteristics of a first element depicted in the third virtual representation is different from one or more corresponding second visual characteristics of the first element depicted in the fourth virtual representation.

10. The system of claim 9, wherein one or more first visual characteristics comprise a first color for the first element depicted and the one or more second visual characteristics comprise a second color for the first element, the second color being different from the first color.

11. The system of claim 9, wherein the one or more first visual characteristics include one or more of a mesh, a transparency, a symbol, or an icon for the first element.

12. The system of claim 9, wherein the first element is the path, the passageway, a target, a border, or a background.

13. The system of claim 8, wherein the memory stores instructions that, when executed by the one or more processors, cause the one or more processors to display a label indicating whether the linking mode is activated or deactivated.

14. The system of any one of claims 1-13, wherein the passageway is a passageway within patient anatomy.

15. One or more non-transitory machine-readable media including a plurality of machine-readable instructions which when executed by one or more processors are adapted to cause the one or more processors to perform a method comprising:
causing display on a display system:
a first virtual representation of a passageway based on a pose of an elongate device, and
a virtual representation of a path associated with the elongate device;
receiving a first input via a first control device;
in response to the first input while the elongate device is being navigated in a linking mode, causing display on the display system:
a second virtual representation of the passageway corresponding to movement along the virtual representation of the path, wherein the movement along the virtual representation of the path is based on the first input;
receiving a second input via a second control device; and
in response to the second input, causing display on the display system:
a third virtual representation of the passageway corresponding to a change to the second virtual representation along at least one of a translational or rotational degree of freedom, wherein the change to the second virtual representation is based on the second input.
